# EUROPEAN PATENT APPLICATION

(11) **EP 3 904 367 A1**
(43) Date of publication of application: **03.11.2021**
(21) Application number: 19905086.5
(22) Date of filing: 20.11.2019
(51) Int. Cl.: C07K 5/103, C07K 5/097, C07K 1/06, C07K 1/02, C12P 17/10

(54) **TETRAPEPTIDE COMPOUND, PREPARATION METHOD AND USE THEREOF**

(30) Priority: 27.12.2018 CN 201811616048
(71) Applicant: Lanzhou University, Lanzhou, Gansu 730000 (CN); Hangzhou Yinfu Science & Technology Co., Ltd., Hangzhou, Zhejiang 311305 (CN)
(72) Inventor: DA, Chaoshan, Lanzhou, Gansu 730000 (CN); DU, Zhihong, Lanzhou, Gansu 730000 (CN); QIN, Wenjuan, Lanzhou, Gansu 730000 (CN); TAO, Baoxiu, Lanzhou, Gansu 730000 (CN); BAI, Yanbing, Hangzhou, Zhejiang 311305 (CN); LIN, Hang, Hangzhou, Zhejiang 311305 (CN); ZHANG, Lianchun, Hangzhou, Zhejiang 311305 (CN); YIN, Hanghua, Hangzhou, Zhejiang 311305 (CN); YU, Jianxin, Hangzhou, Zhejiang 311305 (CN); LIU, Xueyu, Hangzhou, Zhejiang 311305 (CN); JIANG, Weilin, Hangzhou, Zhejiang 311305 (CN); JIN, Wenjiu, Hangzhou, Zhejiang 311305 (CN)
(74) Representative: ABG Intellectual Property Law, S.L.
(86) International application number: PCT/CN2019/119628
(87) International publication number: WO 2020/134727

(57) **Abstract**

The present invention discloses a tetrapeptide compound, a preparation method and a use thereof. The tetrapeptide compound of the present invention is TP represented by formula 1, the preparation method thereof is firstly preparing an intermediate compound, then adding the intermediate compound and catalysts Pd/C into a solvent, introducing hydrogen gas with the pressure being 1 to 30 Mpa for reaction, to obtain a final product of tetrapeptide compound TP. The compound of the present invention can be used for Aldol reaction of ketone and aromatic aldehyde, or can be used for catalyzing asymmetric conjugate addition reaction of fatty aldehyde and maleimide. The tetrapeptide compound of the present invention can be used for catalyzing the conjugate addition reaction of fatty aldehyde and maleimide at high efficiency in an asymmetric way, the reaction yield reaches 98%, the enantioselectivity is as high as 99%.

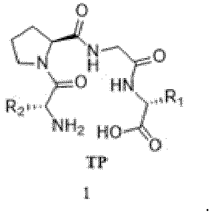

## Description

This application claims the priority to Chinese Patent Application No. 201811616048X, titled "TETRAPEPTIDE COMPOUND, PREPARATION METHOD AND USE THEREOF", filed on December 27, 2018 with the China National Intellectual Property Administration, which is incorporated herein by reference in entirety.

### FIELD

The present disclosure relates to a tetrapeptide compound, a preparation method and application thereof. Sepecifically, the present disclosure relates to a tetrapeptide compound, a preparation method and application thereof, and particularly to a compound that can be applied to catalyze an asymmetric conjugate addition reaction of an aliphatic aldehyde with a maleimide, a preparation method and application thereof.

### BACKGROUND

Optically active compounds are ubiquitous in nature and play an irreplaceable role in the life activities of cells and organisms. Many drugs have optical activity, in which chirality is an important parameter. The most economical and green way to synthesize a chiral compound is to catalyze an asymmetric reaction with a chiral catalyst. Asymmetric catalysis includes three fields, namely, chiral ligand-metal complex catalysts, small organic molecule catalysts and biological enzyme-catalyzed reactions. Owing to its green and cost-effective properties, bioenzyme catalysis has always been a research hotspot in asymmetric catalysis, but due to the structural complexity, easy inactivation, specificity and other limitations of biological enzymes, it has not been quickly applied. Polypeptides are a new type of organocatalysts designed and synthesized according to the enzyme catalytic mechanism, and have many advantages such as simple and stable structures, easy to be synthesized, wide scopes of substrates, applicable to many types of reactions, and mild reaction conditions. They are excellent mimics of natural enzymes, and are the key direction of chemical biology research.

It has been reported that synthetic short peptides are able to well replace natural proteases and mimic enzymes to catalyze a series of chemical reactions, and have been widely used to catalyze asymmetric reactions such as Aldol, Michael, Stetter, azidation, Strecker, Baylis-Hillman, allylic substitution, hydrocyanation, epoxidation, hydrogenation and acylation so as to synthetize a variety of optically active compounds, which has effectively overcome the defects of protease-catalyzed reactions, such as substrate specificity and single reaction type, thereby expanding the type and substrate of the reaction. They are applicable to various reaction conditions, and basically retain the green and economical advantages of biocatalytic reaction. [(1) Aixiang LIU, Yao FU, Lei LIU, Qingxiang GUO, organic chemistry, 2007, 27, 1195-1219. (2) Davie, E. A. C.; Mennen, S. M.; Xu, Y.; Miller, S. J. Chem. Rev. 2007, 107, 5759-5812. (3) Wennemers, H. Chem. Commun., 2011, 47, 12036―12041. (4) Lewandowski, B.; Wennemers, H. Curr. Opin. Chem. Biol. 2014, 22, 40―46. (5) Kelly, D. R.; Roberts, S. M. Biopolymers 2006, 84, 74―89. (6) Akagawa, K.; Kudo, K. Acc. Chem. Res. 2017, 50, 2429-2439. (7) Ball, Z. T. Acc. Chem. Res. 2012, 46, 560―570.]. Therefore, to discover new and efficient short peptide catalysts that mimic proteases to catalyze organic chemical reactions has been a hot spot in the field of synthesis of chiral compounds.

The chiral 3-substituted succinimides synthesized under asymmetric catalytic conditions by the asymmetric conjugate addition reactions of nucleophiles with *N*-substituted maleimides, may be easily converted, through reduction, into a series of chiral pyrrole compounds, which are the core frameworks of many bioactive compounds, and so the asymmetric synthesis of chiral pyrrole compounds has been a core subject in organic chemistry and medicinal chemistry researches. In addition, the chiral 3-substituted succinimides may also be easily converted into chiral succinic acid through hydrolysis reactions, so it is an important physiologically active compound and a chiral synthetic building block. As the product from asymmetric conjugated addition of nucleophiles to maleimides, an aldehyde additionally has an active group that is readily converted into other functional groups, so that such reactions have important application value. Therefore, it is a goal pursued by many research institutions to continuously develop a highly efficient chiral catalyst for catalyzing the asymmetric conjugate addition reaction of an aliphatic aldehyde with a maleimide. To summarize the literature reports, it can be seen that the catalysts successfully applied to this type of reaction are almost all chiral organocatalysts, most of which are primary amine-thioureas or squaramides-based catalysts derived from chiral cyclohexanediamine as raw materials, and have achieved desired yields and enantioselectivities. [(8)Xue, F.; Liu, L.; Zhang, S.; Duan, W.; Wang, W. Chem. Eur. J. 2010, 16, 7979-7982; (9) Ma, Z.-W.; Liu, Y.-X.; Zhang, W.-J.; Tao, Y.; Zhu, Y.; Tao, J.-C.; Tang, M.-S. Eur. J. Org. Chem. 2011, 6747-6754. (10) Orlandi, S.; Pozzi, G.; Ghisetti, M.; Benaglia, M. New J. Chem. 2013, 37, 4140-4147. (11) Yu, F.; Jin, Z.; Huang, H.; Ye, T.; Liang, X.; Ye, J. Org. Biomol. Chem. 2010, 8, 4767-4774. (12) Bai, J.-F.; Peng, L.; Wang, L.-L.; Wang, L.-X.; Xu, X.-Y. Tetrahedron 2010, 66, 8928-8932. (13) Ma, Z.-W.; Liu, Y.-X.; Li, P.-L.; Ren, H.; Zhu, Y.; Tao, J.-C.; Tetrahedron: Asymmetry 2011, 22, 1740-1748. (14) Ma, Z.-W.; Liu, X.-F.; Liu, J.-T.; Liu, Z.-J.; Tao, J.-C. Tetrahedron Letters 2017, 58, 4487-4490.]. Cinchona alkaloid thiourea and 2-chlorophenylglycine being an efficient dual-catalyst system also achieved desired yields and enantioselectivities. [(15) Muramulla, S.; Ma, J.-A.; Zhao, J. C.-G. Adv. Synth. Catal. 2013, 355, 1260-1264]. With the addition of triphenylphosphine, cinchonidine-derived primary amine exhibited excellent catalytic yields and enantioselectivities [(16) Yang, W.; Jiang, K.-Z.; Lu, X.; Yang, H.-M.; Li, L.; Lu, Y.; Xu, L.-W. Chem. Asian J. 2013, 8, 1182-1190]. It was found that with the addition of alkali metals, using β-phenylalanine and α-tert-butyl aspartate to catalyze such reactions, or using side chain tert-butyl-protected threonine and isoleucine, both achieved good asymmetric catalytic effects [(17) Kokotos, C. G. Org. Lett. 2013, 15, 2406-2409. (18) Nugent, T. C.; Sadiq, A.; Bibi, A.; Heine, T.; Liu L.; Vankova, N.; Bassil, B. S. Chem. Eur. J. 2012, 18, 4088-4098]. There was also one report on dipeptide Ala-Ala catalyzed reactions between propionaldehyde and maleimide, but the ee value of its product was only 38% [(19) Zhao, G.-L.; Xu, Y.; Sundén, H.; Eriksson, L.; Sayah, M.; Córdova, A. Chem. Commun. 2007, 734-735.]. Therefore, it is a challenging task to develop high-efficiency polypeptide catalysts for such a reaction.

### SUMMARY

The present disclosure provides a tetrapeptide compound, a preparation method and application thereof. The tetrapeptide compounds provided by the present disclosure can efficiently and asymmetrically catalyze the conjugate addition reaction of an aliphatic aldehyde with a maleimide, which have overcome the shortcomings of the prior art.

The tetrapeptide compounds described in the present disclosure are represented by **TP** as shown in Formula 1, 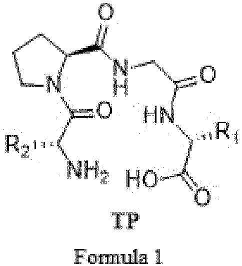 wherein each of R₁ and R₂ is any one of any C₁-C₆ straight-chain alkyl, branched-chain alkyl, cycloalkyl, hydroxy-substituted alkyl, sulfhydryl-substituted alkyl, methylthio-substituted alkyl, amino-substituted alkyl, guanidyl-substituted alkyl, aryl, arylmethyl or heteroaryl.

Preferably, in the tetrapeptide compound described in the present disclosure, each of R₁ and R₂ is any one of any C₁-C₄ straight-chain alkyl, branched-chain alkyl, cyclohexyl, phenyl, and benzyl.

More preferably, for the tetrapeptide compounds described in the present disclosure, when R₁ = *i*-Bu, and R₂ = *i*-Pr, the amino acid sequence thereof is H₂N-D-Val-Pro-Gly-D-Leu-OH which is named **TP-1**.

Or, for the tetrapeptide compounds described in the present disclosure, when R₁ = *i*-Bu, and R₂ = *t*-Bu, the amino acid sequence thereof is H₂N-D-Tle-Pro-Gly-D-Leu-OH which is named **TP-2**.

Or, for the tetrapeptide compounds described in the present disclosure, when R₁ = *i*-Bu, and R₂ = Ph, the amino acid sequence thereof is H₂N-D-Phg-Pro-Gly-D-Leu-OH which is named **TP-3**.

Or, for the tetrapeptide compounds described in the present disclosure, when R₁ = *i*-Bu, and R₂ = c-hex, the amino acid sequence thereof is H₂N-D-Chg-Pro-Gly-D-Leu-OH which is named **TP-4**.

Or, for the tetrapeptide compounds described in the present disclosure, when R₁ =*i*-Bu, and R₂ = Bn, the amino acid sequence thereof is H₂N-D-Phe-Pro-Gly-D-Leu-OH which is named **TP-5**.

Or, for the tetrapeptide compounds described in the present disclosure, when R₁ = Bn, and R₂ = Bn, the amino acid sequence thereof is H₂N-D-Phe-Pro-Gly-D-Phe-OH which is named **TP-6**.

The method of preparing the tetrapeptide compounds of the present disclosure is performed by carrying out reactions as shown in Formula 2, Formula 3, Formula 4 and Formula 5, that is: 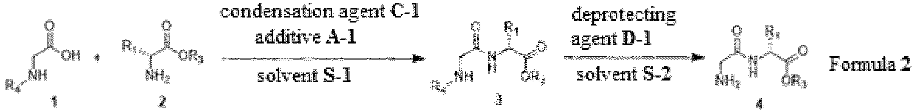 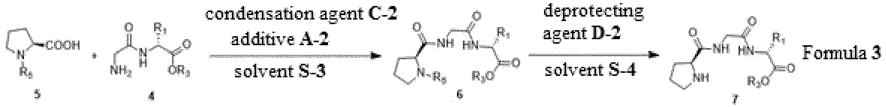 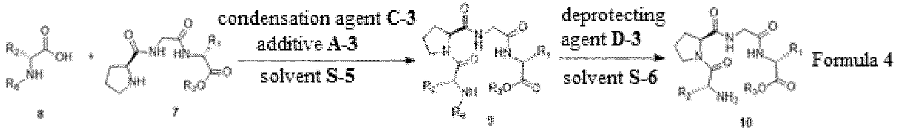 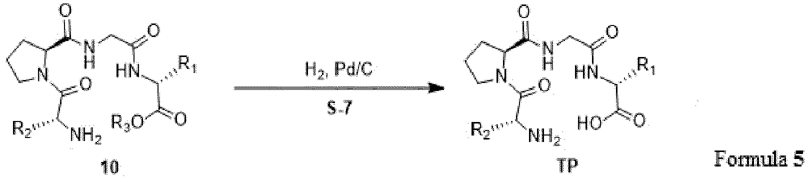
adding glycine **1**, an amino acid **2**, a condensation agent **C-1** and an additive **A-1** into a solvent **S-1** and stirring to react to obtain a product **3,** then adding the product **3** and a deprotecting agent **D-1** into a solvent **S-2** and stirring to react to obtain a product **4**;
adding the product **4**, proline **5**, a condensation agent **C-2** and an additive **A-2** into a solvent **S-3** and stirring to react to obtain a product **6**, then adding the product **6** and a deprotecting agent **D-2** into a solvent **S-4** and stirring to react to obtain a product 7;
adding the product **7**, an amino acid **8**, a condensation agent **C-3** and an additive **A-3** into a solvent **S-5** and stirring to react to obtain a product **9**, then adding the product **9** and a deprotecting agent **D-3** into a solvent **S-6** and stirring to react to obtain a product **10**;
adding the product **10** and a catalyst Pd/C into a solvent **S-7**, to react with hydrogen introduced at a pressure of 1-30 Mpa, to obtain a final product tetrapeptide **TP**, wherein
R₃ is arylmethyl;
R₄, R₅ and R₆ are any one of Boc (tert-butyloxycarbonyl), Cbz (benzyloxycarbonyl) or Fmoc (fluorenylmethoxycarbonyl);
condensation agents **C-1**, **C-2** and **C-3** are any one of carbodiimides, or alkoxyformyl chlorides (R₇OCOCl), and R₇ is any C₁-C₄ straight-chain alkyl or branched-chain alkyl;
additives **A-1**, **A-2** and **A-3** refer to R₈R₉R₁₀N or NMM (N-methylmorpholine) or TMEDA (4-dimethylaminopyridine) or HOBt(1-hydroxybenzotriazole), and R₈, R₉ and R₁₀ are any C₁-C₄ straight-chain alkyl or branched-chain alkyl;
solvents **S-1**, **S-2, S-3**, **S-4**, **S-5** and **S-6** are any one of toluene, benzene, dichloromethane, dichloroethane, ethyl ether, tetrahydrofuran, ethylene glycol dimethyl ether, methyl *tert-*butyl ether, dioxane, ethyl acetate, methyl acetate, acetonitrile or propionitrile, or a combination thereof, and solvent **S-7** is any one of methanol, ethanol, propanol or butanol, or a combination thereof; and
deprotecting agents **D-1**, **D-2** and **D-3** are any one of methanesulfonic acid, trifluoromethanesulfonic acid, benzenesulfonic acid, *para*-toluenesulfonic acid, trifluoroacetic acid, trichloroacetic acid, hydrochloric acid, sulfuric acid, piperidine, morpholine, tetrahydropyrrole, dihydropyrrole, pyrrole, diethylamine, dipropylamine, dibutylamine, diisopropylamine or diisobutylamine.

Preferably, in the preparation method of the compounds described in the present disclosure:
R₃ is benzyl;
R₄, R₅ and R₆ refer to Boc or Cbz;
condensation agents **C-1**, **C-2** and **C-3** are DCC (N,N'-dicyclohexylcarbodiimide) or DIC (N,N'-diisopropylcarbodiimide), or an alkoxyformyl chloride R₇OCOCl, and R₇ is Et or *i*-Pr or *i*-Bu;
additives **A-1**, **A-2** and **A-3** are any one of TEA (triethylamine), DIPEA (diisopropylethylamine), NMM or HOBt;
solvents **S-1**, **S-2**, **S-3**, **S-4**, **S-5** and **S-6** are dichloromethane or tetrahydrofuran, and solvent S-7 is methanol or ethanol; and
deprotecting agents **D-1**, **D-2** and **D-3** are methanesulfonic acid, or trifluoromethanesulfonic acid or trifluoroacetic acid.

Preferably, in the preparation method of the compounds described in the present disclosure:
R₃ is benzyl;
R₄, R₅ and R₆ are Boc;
condensation agents **C-1**, **C-2** and **C-3** are DCC or DIC or an alkoxyformyl chloride R₇OCOCl, and R₇ is Et or *i*-Pr or *i*-Bu;
additives **A-1**, **A-2** and **A-3** refer to any one of TEA, DIPEA, NMM or HOBt;
solvents **S-1**, **S-2**, **S-3**, **S-4**, **S-5** and **S-6** refer to dichloromethane or tetrahydrofuran, and solvent **S-7** refers to methanol;
deprotecting agents **D-1**, **D-2** and **D-3** refer to trifluoroacetic acid; and

The pressure of hydrogen gas is 8 Mpa.

The compounds described in the present disclosure may be applied to the Aldol reaction of a ketone with an aromatic aldehyde, or be used to catalyze an asymmetric conjugate addition reaction of an aliphatic aldehyde with a maleimide as shown in Formula 6. That is, as shown in Formula 6, an aliphatic aldehyde **11**, a maleimide **12** and the tetrapeptide **TP** are added into a reaction vessel containing a solvent **S-8** and stirred to obtain 3-substituted succinimide (*R*)-**P** as the product from the conjugate addition reaction, 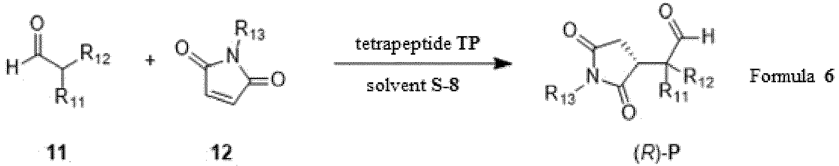
wherein R₁₁ is hydrogen or any C₁-C₆ straight-chain alkyl or branched-chain alkyl or phenyl or benzyl, and R₁₂ is any C₁-C₆ straight-chain alkyl or branched-chain alkyl or phenyl or benzyl; or -R₁₁-R₁₂- is cyclohexyl or cyclopentyl; and R₁₃ is hydrogen or phenyl or substituted phenyl or straight-chain alkyl or cycloalkyl or arylmethyl;
tetrapeptide **TP** is a tetrapeptide containing D-configuration-α-primary amino acids at both terminals as shown in Formula 1;
In the above reaction, solvent **S-8** refer to any one or more of dichloromethane, chloroform, dichloroethane, ethyl acetate, methyl acetate, toluene, benzene, xylene, ethyl ether, tetrahydrofuran, ethylene glycol dimethyl ether, methyl tert-butyl ether, acetonitrile, propionitrile, ethanol, methanol, propanol, and butanol.

Preferably:
R₁₁ is hydrogen or methyl or ethyl, R₁₂ is methyl or ethyl or isopropyl or *n*-butyl; or -R₁₁-R₁₂- is cyclohexyl or cyclopentyl; R₁₃ is hydrogen or methyl or cyclohexyl or benzyl or phenyl or substituted phenyl, and the substituted phenyl herein refers to *para*-methylphenyl or *para*-chlorophenyl or *para*-bromophenyl or *para*-fluorophenyl or *para*-nitrophenyl or *para*-methoxyphenyl;
tetrapeptide **TP** is any one of **TP-1** to **TP-6** described above; and
solvent **S-8** is any one of dichloromethane, acetonitrile, ethanol, tetrahydrofuran, dimethyl sulfoxide, or toluene.

More preferably, tetrapeptide **TP** is **TP-3**; and solvent S-8 is acetonitrile.

In the present disclosure, an intermediate compound for use in preparation of a compound **TP** represented by Formula 1 is the compound **6** as shown in Formula 3, or the compound 7 as shown in Formula 3, or the compound **9** as shown in Formula 4, or the compound **10** as shown in Formula 4.

The compounds of the present disclosure can catalyze the conjugate addition reaction of an aliphatic aldehyde with a maleimide with high yields and high enantioselectivities during the asymmetrically catalyzed conjugation reaction of the aliphatic aldehyde and the maleimide. According to related experiments, the tetrapeptide compounds of the present invention can efficiently and asymmetrically catalyze the conjugate addition reaction of an aliphatic aldehyde with a maleimide, with a reaction yield up to 98% and an enantioselectivity up to 99%, which shows a good application prospect.

### DETAILED DESCRIPTION

In order to enable those skilled in the art to well understand the technical solutions of this disclosure, this disclosure will be further described in detail below in conjunction with specific embodiments.

The present disclosure provides a tetrapeptide compound **TP** represented by Formula 1, 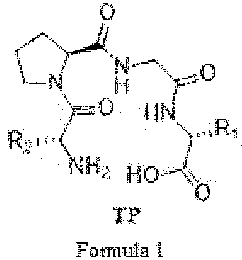 wherein R₁ and R₂ are independently any one selected from any C₁-C₆ straight-chain alkyl or branched-chain alkyl, cycloalkyl, hydroxy-substituted alkyl, sulfhydryl-substituted alkyl, methylthio-substituted alkyl, amino-substituted alkyl, guanidyl-substituted alkyl, aryl, arylmethyl or heteroaryl.

In accordance with the present disclosure, R₁ is selected from any C₁-C₆ straight-chain alkyl, C₃-C₁₅ branched-chain alkyl, C₃-C₈ cycloalkyl, hydroxyl-substituted C₁-C₆ alkyl, sulfhydryl substituted C₁-C₆ alkyl, methylthio-substituted C₁-C₆ alkyl, amino-substituted C₁-C₆ alkyl, guanidyl-substituted C₁-C₆ alkyl, C₆-C₃₀ aryl, C₆-C₃₀ arylmethyl or C₅-C₁₅ heteroaryl, and preferably is methyl, ethyl, *n*-propyl, isopropyl, *n*-butyl, isobutyl, *t*-butyl, *n*-pentyl, isoamyl, *n*-hexyl, cyclopropyl, cyclobutyl, cyclopentyl, cyclohexyl, cycloheptyl, hydroxymethyl, 2-hydroxyethyl, 1-hydroxyethyl (MeCH(OH)-), aminomethyl, 2-aminoethyl, 3-amino *n*-propyl, 4-amino *n*-butyl, mercaptomethyl, 2-mercaptoethyl, methylthiomethyl, 2-methylthioethyl, phenyl, naphthyl, anthryl, phenanthryl, furyl, pyridyl, pyranyl, piperidinyl, phenylmethyl, naphthylmethyl or anthrylmethyl.

R₂ is selected from any C₁-C₆ straight-chain alkyl, C₃-C₁₅ branched-chain alkyl, C₃-C₈ cycloalkyl, hydroxyl-substituted C₁-C₆ alkyl, sulfhydryl substituted C₁-C₆ alkyl, methylthio-substituted C₁-C₆ alkyl, amino-substituted C₁-C₆ alkyl, guanidyl-substituted C₁-C₆ alkyl, C₆-C₃₀ aryl, C₆-C₃₀ arylmethyl or C₅-C₁₅ heteroaryl, and preferably is methyl, ethyl, *n*-propyl, isopropyl, *n*-butyl, isobutyl, *t*-butyl, *n*-pentyl, isoamyl, *n*-hexyl, cyclopropyl, cyclobutyl, cyclopentyl, cyclohexyl, cycloheptyl, hydroxymethyl, 2-hydroxyethyl, 1-hydroxyethyl (MeCH(OH)-), aminomethyl, 2-aminoethyl, 3-amino *n*-propyl, 4-amino *n*-butyl, mercaptomethyl, 2-mercaptoethyl, methylthiomethyl, 2-methylthioethyl, phenyl, naphthyl, anthryl, phenanthryl, furyl, pyridyl, pyranyl, piperidinyl, phenylmethyl, naphthylmethyl or anthrylmethyl.

More specifically, in the tetrapeptide compound **TP** having the structure of Formula (1),
R₁ is *i*-Bu, and R₂ is *i*-Pr, that is, the tetrapeptide compound **TP** has an amino acid sequence of H₂N-D-Val-Pro-Gly-D-Leu-OH which is named **TP-1**;
R₁ is *i*-Bu, and R₂ is *t*-Bu, that is, the tetrapeptide compound **TP** has an amino acid sequence of H₂N-D-Tle-Pro-Gly-D-Leu-OH which is named **TP-2**;
R₁ is *i*-Bu, and R₂ is Ph, that is, the tetrapeptide compound **TP** has an amino acid sequence of H₂N-D-Phg-Pro-Gly-D-Leu-OH which is named **TP-3**;
R₁ is *i*-Bu, and R₂ is c-hex, that is, the tetrapeptide compound **TP** has an amino acid sequence of H₂N-D-Chg-Pro-Gly-D-Leu-OH which is named **TP-4**;
R₁ is *i*-Bu, and R₂ is Bn, that is, the tetrapeptide compound TP has an amino acid sequence of H₂N-D-Phe-Pro-Gly-D-Leu-OH which is named **TP-5**; and
R₁ is Bn, and R₂ is Bn, that is, the tetrapeptide compound **TP** has an amino acid sequence of H₂N-D-Phe-Pro-Gly-D-Phe-OH which is named **TP-6**.

The present disclosure further provides a preparation method of the tetrapeptide compound **TP** represented by Formula 1 described in the present disclosure, which comprises:
1) mixing and reacting a product 7 with an amino acid **8**, to obtain a product **9**; 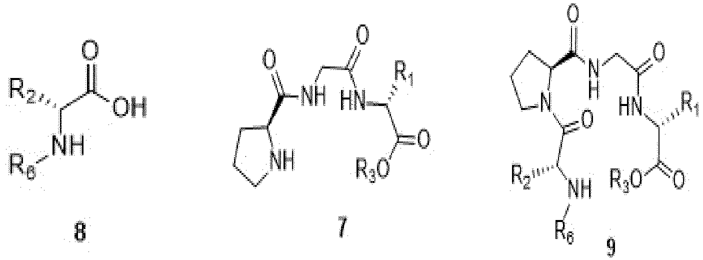
   wherein R₁ and R₂ are independently any one selected from any C₁-C₆ straight-chain alkyl or branched-chain alkyl, cycloalkyl, hydroxy-substituted alkyl, sulfhydryl-substituted alkyl, methylthio-substituted alkyl, amino-substituted alkyl, guanidyl-substituted alkyl, aryl, arylmethyl or heteroaryl;
   R₃ is arylmethyl;
   R₆ is selected from *tert*-butyloxycarbonyl, benzyloxycarbonyl or fluorenylmethoxycarbonyl;
2) converting the product **9** into a tetrapeptide compound **TP** represented by Formula 1;

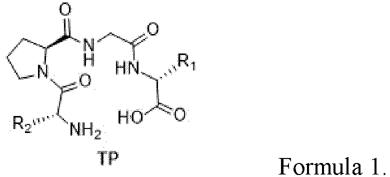

In accordance with the present disclosure, a product **7** is mixed and reacted with an amino acid **8** to obtain a product **9** in the present disclosure, in which the condensation agent in the condensation reaction is an carbodiimide, or an alkoxyformyl chloride (R₇OCOCl), wherein R₇ is any C₁-C₄ straight-chain alkyl or branched-chain alkyl; the additive in the reaction is R₈R₉R₁₀N, NMM (N-methylmorpholine), TMEDA (4-dimethylaminopyridine) or HOBt (1-hydroxybenzotriazole), wherein R₈, R₉ and R₁₀ are independently selected from any C₁-C₄ straight-chain alkyl or branched-chain alkyl; the solvent for the reaction is preferably one or more of toluene, benzene, dichloromethane, dichloroethane, ethyl ether, tetrahydrofuran, ethylene glycol dimethyl ether, methyl *tert*-butyl ether, dioxane, ethyl acetate, methyl acetate, acetonitrile and propionitrile.

In the present disclosure, there are no special requirements on the source of product 7 of the present disclosure. Preferably, the product 7 is prepared by the following way:

A-1) reacting a product **4** with proline **5** to obtain a product **6*,*** in which the condensation agent in the reaction is an carbodiimide, or an alkoxyformyl chloride (R₇OCOCl), wherein R₇ is any C₁-C₄ straight-chain alkyl or branched-chain alkyl; the additive in the reaction is R₈R₉R₁₀N, NMM (N-methylmorpholine), TMEDA (4-dimethylaminopyridine) or HOBt (1-hydroxybenzotriazole), wherein R₈, R₉ and R₁₀ are independently selected from any C₁-C₄ straight-chain alkyl or branched-chain alkyl; the solvent for the reaction is preferably one or more of toluene, benzene, dichloromethane, dichloroethane, ethyl ether, tetrahydrofuran, ethylene glycol dimethyl ether, methyl *tert*-butyl ether, dioxane, ethyl acetate, methyl acetate, acetonitrile and propionitrile; 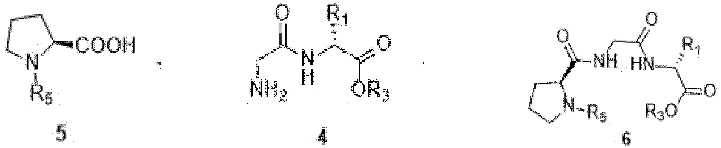
wherein, R₁ is any one selected from any C₁-C₆ straight-chain alkyl or branched-chain alkyl, cycloalkyl, hydroxy-substituted alkyl, sulfhydryl-substituted alkyl, methylthio-substituted alkyl, amino-substituted alkyl, guanidyl-substituted alkyl, aryl, arylmethyl or heteroaryl;
R₃ is arylmethyl;
R₅ is selected from *tert*-butyloxycarbonyl, benzyloxycarbonyl or fluorenylmethoxycarbonyl;
A-2) converting the product **6** into the product **7,** in which the deprotecting agent in the conversion reaction is preferably methanesulfonic acid, trifluoromethanesulfonic acid, benzenesulfonic acid, *para*-toluenesulfonic acid, trifluoroacetic acid, trichloroacetic acid, hydrochloric acid, sulfuric acid, piperidine, morpholine, tetrahydropyrrole, dihydropyrrole, pyrrole, diethylamine, dipropylamine, dibutylamine, diisopropylamine or diisobutylamine; the solvent for the reaction is preferably one or more of toluene, benzene, dichloromethane, dichloroethane, ethyl ether, tetrahydrofuran, ethylene glycol dimethyl ether, methyl tert-butyl ether, dioxane, ethyl acetate, methyl acetate, acetonitrile and propionitrile.

In the present disclosure, the product **4** is prepared by the following way:
B-1) mixing and reacting a glycine **1** with an amino acid **2** to obtain a product 3, in which the condensation agent in the reaction is an carbodiimide, or an alkoxyformyl chloride R₇OCOCl, wherein R₇ is any C₁-C₄ straight-chain alkyl or branched-chain alkyl; the additive in the reaction is R₈R₉R₁₀N, NMM (N-methylmorpholine), TMEDA (4-dimethylaminopyridine) or HOBt(1-hydroxybenzotriazole), wherein R₈, R₉ and R₁₀ are independently selected from any C₁-C₄ straight-chain alkyl or branched-chain alkyl; the solvent in the reaction is preferably one or more toluene, benzene, dichloromethane, dichloroethane, ethyl ether, tetrahydrofuran, ethylene glycol dimethyl ether, methyl tert-butyl ether, epoxyhexane, ethyl acetate, methyl acetate, acetonitrile and propionitrile; 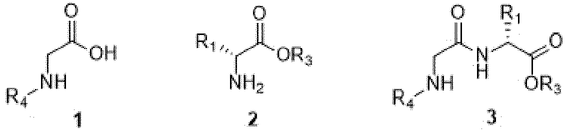
wherein, R₁ is any one selected from any C₁-C₆ straight-chain alkyl, branched-chain alkyl, cycloalkyl, hydroxy-substituted alkyl, sulfhydryl-substituted alkyl, methylthio-substituted alkyl, amino-substituted alkyl, guanidyl-substituted alkyl, aryl, arylmethyl or heteroaryl;
R₃ is arylmethyl;
R₄ and R₅ are independently selected from *tert*-butyloxycarbonyl, benzyloxycarbonyl or fluorenylmethoxycarbonyl;
B-2) converting the product **3** into the product **4**, in which the deprotecting agent in the conversion reaction is preferably methanesulfonic acid, trifluoromethanesulfonic acid, benzenesulfonic acid, *para*-toluenesulfonic acid, trifluoroacetic acid, trichloroacetic acid, hydrochloric acid, sulfuric acid, piperidine, morpholine, tetrahydropyrrole, dihydropyrrole, pyrrole, diethylamine, dipropylamine, dibutylamine, diisopropylamine or diisobutylamine; and the solvent for the reaction is preferably one or more of toluene, benzene, dichloromethane, dichloroethane, ethyl ether, tetrahydrofuran, ethylene glycol dimethyl ether, methyl *tert*-butyl ether, dioxane, ethyl acetate, methyl acetate, acetonitrile and propionitrile.

In accordance with the present disclosure, the step 2) is specifically as following:
deprotecing the product **9** to obtain a product **10**, in which the deprotecting agent for deprotection is preferably methanesulfonic acid, trifluoromethanesulfonic acid, benzenesulfonic acid, *para*-toluenesulfonic acid, trifluoroacetic acid, trichloroacetic acid, hydrochloric acid, sulfuric acid, piperidine, morpholine, tetrahydropyrrole, dihydropyrrole, pyrrole, diethylamine, dipropylamine, dibutylamine, diisopropylamine or diisobutylamine; and the solvent for the reaction is preferably one or more of toluene, benzene, dichloromethane, dichloroethane, ethyl ether, tetrahydrofuran, ethylene glycol dimethyl ether, methyl tert-butyl ether, epoxyhexane, ethyl acetate, methyl acetate, acetonitrile and propionitrile; 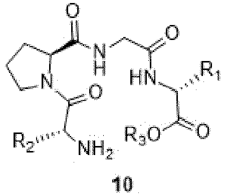
wherein R1, and R₂ are independently any one selected from any C₁-C₆ straight-chain alkyl or branched-chain alkyl, cycloalkyl, hydroxy-substituted alkyl, sulfhydryl-substituted alkyl, methylthio-substituted alkyl, amino-substituted alkyl, guanidyl-substituted alkyl, aryl, arylmethyl or heteroaryl;
R₃ is arylmethyl; and
R₆ is selected from *tert*-butyloxycarbonyl, benzyloxycarbonyl or fluorenylmethoxycarbonyl; and
subjecting the product **10** to a catalytic hydrogenation reaction to obtain a tetrapeptide compound **TP** represented by Formula 1.

More specifically, the preparation method of the tetrapeptide compound **TP** represented by Formula 1 is specifically as follows: carring out reaction schemes as shown in Formula 2, Formula 3, Formula 4 and Formula 5, that is: 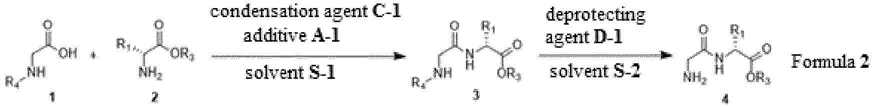 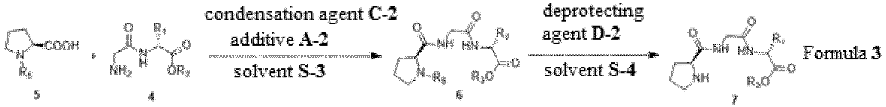 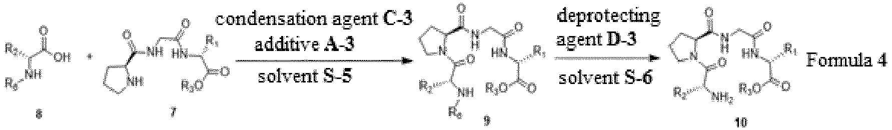 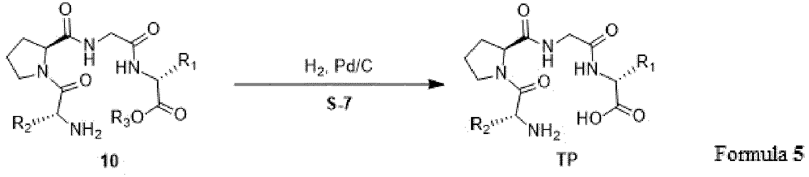
adding glycine **1**, an amino acid **2**, a condensation agent **C-1** and an additive **A-1** into a solvent **S-1** and stirring to react to obtain a product **3**, then adding the product **3** and a deprotecting agent **D-1** into a solvent **S-2** and stirring to react to obtain a product **4**;
adding the product **4**, proline **5**, a condensation agent **C-2** and an additive **A-2** into a solvent **S-3** and stirring to react to obtain a product **6**, then adding the product **6** and a deprotecting agent **D-2** into a solvent **S-4** and stirring to react to obtain a product 7;
adding the product **7**, an amino acid **8**, a condensation agent **C-3** and an additive **A-3** into a solvent **S-5** and stirring to react to obtain a product **9**, then adding the product **9** and a deprotecting agent **D-3** into a solvent **S-6** and stirring to react to obtain a product **10**; and
adding the product **10** and a catalyst Pd/C into a solvent **S-7** with hydrogen gas introduced at a pressure of 1-30 Mpa to react to obtain a final product tetrapeptide **TP**, in which
R₃ is arylmethyl;
R₄, R₅ and R₆ are any one of Boc (tert-butyloxycarbonyl), Cbz (benzyloxycarbonyl) or Fmoc (fluorenylmethoxycarbonyl);
condensation agents **C-1**, **C-2** and **C-3** are any one of carbodiimides, or alkoxyformyl chlorides (R₇OCOCl), and R₇ is any C₁-C₄ straight-chain alkyl or branched-chain alkyl;
additives **A-1**, **A-2** and **A-3** refer to R₈R₉R₁₀N or NMM (N-methylmorpholine) or TMEDA (4-dimethylaminopyridine) or HOBt (1-hydroxybenzotriazole), and R₈, R₉ and R₁₀ are any C₁-C₄ straight-chain alkyl or branched-chain alkyl;
solvents **S-1**, **S-2**, **S-3**, **S-4**, **S-5** and **S-6** are any one of toluene, benzene, dichloromethane, dichloroethane, ethyl ether, tetrahydrofuran, ethylene glycol dimethyl ether, methyl tert-butyl ether, dioxane, ethyl acetate, methyl acetate, acetonitrile or propionitrile, or a combination thereof; and solvent **S-7** is any one of methanol, ethanol, propanol or butanol, or a combination thereof;
deprotecting agents **D-1**, **D-2** and **D-3** are any one of methanesulfonic acid, trifluoromethanesulfonic acid, benzenesulfonic acid, *para*-toluenesulfonic acid, trifluoroacetic acid, trichloroacetic acid, hydrochloric acid, sulfuric acid, piperidine, morpholine, tetrahydropyrrole, dihydropyrrole, pyrrole, diethylamine, dipropylamine, dibutylamine, diisopropylamine or diisobutylamine.

In a preferred embodiment of the present disclosure,
R₃ is benzyl;
R₄, R₅ and R₆ are independently selected from Boc or Cbz;
condensation agents **C-1**, **C-2** and **C-3** independently selected from DCC (N,N'-dicyclohexylcarbodiimide) or DIC (N,N'-diisopropylcarbodiimide) or an alkoxyformyl chloride R₇OCOCl,
wherein R₇ is Et or *i*-Pr or *i*-Bu;
additives **A-1**, **A-2** and **A-3** are independently any one selected from TEA (triethylamine), DIPEA (diisopropylethylamine), NMM or HOBt;
solvent **S-1**, **S-2**, **S-3**, **S-4**, **S-5** and **S-6** are independently selected from dichloromethane or tetrahydrofuran, and solvent **S-7** is selected from methanol or ethanol;
deprotecting agents **D-1**, **D-2** and **D-3** are independently selected from methanesulfonic acid, or trifluoromethanesulfonic acid or trifluoroacetic acid.
In another preferred embodiment of the present disclosure,
R₃ is benzyl;
R₄, R₅ and R₆ are independently Boc;
condensation agents **C-1**, **C-2** and **C-3** are independently selected from DCC, DIC or an alkoxyformyl chloride R₇OCOCl, wherein R₇ is selected from Et or *i*-Pr or *i*-Bu;
additives **A-1**, **A-2** and **A-3** are independently any one selected from TEA, DIPEA, NMM or HOBt;
solvents **S-1**, **S-2**, **S-3**, **S-4**, **S-5** and **S-6** are independently selected from dichloromethane or tetrahydrofuran, and solvent **S-7** is methanol;
deprotecting agents **D-1**, **D-2** and **D-3** are independently selected from trifluoroacetic acid; and

The pressure of hydrogen gas is 8 Mpa.

The present disclosure further provides a catalyst for catalyzing an asymmetric conjugate addition reaction of an aliphatic aldehyde with a maleimide, in which the catalyst is a tetrapeptide compound **TP** described in the present disclosure represented by Formula 1; the aliphatic aldehyde has a structure as shown in the compound **11**; the maleimide has a structure as shown in the compound **12**; the product of the asymmetric conjugate addition has a structure as shown in (*R*)**-P;** and the solvent for the conjugate addition reaction is dichloromethane, chloroform, dichloroethane, ethyl acetate, methyl acetate, toluene, benzene, xylene, ethyl ether, tetrahydrofuran, ethylene glycol dimethyl ether, methyl tert-butyl ether, acetonitrile, propionitrile, ethanol, methanol, propanol or butanol.

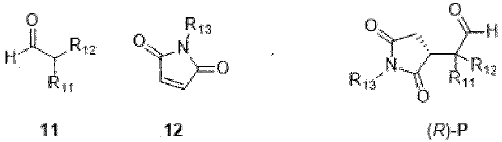

R₁₁ is hydrogen, any C₁-C₆ straight-chain alkyl or branched-chain alkyl, phenyl or benzyl, and more preferably hydrogen, methyl or ethyl; R₁₂ is any C₁-C₆ straight-chain alkyl, branched-chain alkyl, phenyl or benzyl, and preferably methyl, ethyl, isopropyl or *n*-butyl; or -R₁₁-R₁₂- is cyclohexyl or cyclopentyl; R₁₃ is hydrogen, phenyl, substituted phenyl, straight-chain alkyl, cycloalkyl or arylmethyl, and preferably hydrogen, methyl, cyclohexyl, benzyl, phenyl or substituted phenyl, and the substituted phenyl herein refers to *para*-methylphenyl, *para*-chlorophenyl, *para*-bromophenyl, *para*-fluorophenyl, *para*-nitrophenyl or *para*-methoxyphenyl.

The present disclosure further provides an intermediate compound for use in preparation of a compound **TP** represented by Formula 1, which has a structure of compound **6** as shown in Formula 3.

The present disclosure further provides an intermediate compound for use in preparation of a compound **TP** represented by Formula 1, which has a structure of compound 7 as shown in Formula 3.

The present disclosure further provides an intermediate compound for use in preparation of a compound **TP** represented by Formula 1, which has a structure of compound 9 as shown in Formula 4.

The present disclosure further provides an intermediate compound for use in preparation of a compound **TP** represented by Formula 1, which has a structure of compound **10** as shown in Formula 4.

The compounds of the present disclosure can catalyze the conjugate addition reaction of an aliphatic aldehyde with a maleimide with high yields and high enantioselectivities during the asymmetrically catalyzed conjugation reaction of the fatty aldehyde and the maleimide. According to related experiments, the tetrapeptide compounds of the present invention can efficiently and asymmetrically catalyze the conjugate addition reaction of an aliphatic aldehyde with a maleimide, with a reaction yield up to 98%, and an enantioselectivity up to 99%, which shows a good application prospect.

The present disclosure has been explanined in detail above. Hereinafter, the application will be further described in detail in conjunction with examples. However, it is necessary to point out that the following specific embodiments are intended to further illustrate this application, but should not be constructed as limiting the scope of protection of the application. It is possible for those skilled in the art can make some to non-essential improvement and adjustment to this application based on the content of the application described above.

### Example 1

### I. Preparation of tetrapeptide compounds TP

With respect to the tetrapeptide compounds **TP** represented by Formula 1 of the present disclosure, one may refer to formulas 2 to 5 mentioned above for the reaction schemes in their preparation, to the summary section for their preparation methods, and to the following for more details about their preparation.

### (1) Synthesis of dipeptides

### 1.1 Boc-Gly-D-Leu-OBn (3a)

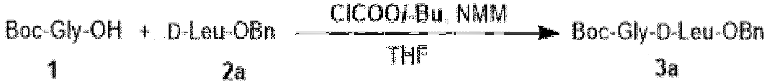

Referring to the above formula, Boc-Gly-OH **1** (8.75 g, 50 mmol) was added into 100 mL of anhydrous THF, and stirred for 5 min under the protection of argon gas in an ice-salt bath, to lower the temperature of the reaction liquid down to -15 °C. After adding NMM (6.2 mL, 55 mmol), **isobutyl chloroformate** (7 mL, 55 mmol) was slowly added dropwise into the reaction solution, and stirred for 5 min. Then, the solution of D-Leu-OBn **2a** *p*-toluenesulphonate (19.7 g, 50 mmol) and NMM (6.2 mL) in anhydrous DMF was added, and stirred continuously at -15 °C to react for 0.5 h. The reaction mixture was naturally warmed to room temperature with the ice-salt bath removed, and the reaction was continued overnight. After the reaction was completed, the insoluble was removed by suction filtration. The filtrate was concentrated under reduced pressure, and then dissolved in ethyl acetate. The ethyl acetate phase was washed successively with 1 M NaOH solution, water, 1 M hydrochloric acid solution and a small amount of saturated brine, dried with anhydrous sodium sulfate, and concentrated under reduced pressure. 19 g of Boc-Gly-D-Leu-OBn (3a), a light yellow oily liquid, was obtained with a yield of 100%. [α]_{D}²⁰ = + 5.0 (c 1.0, CHCl₃); ¹H NMR (200 MHz, CDCl₃) δ 7.34 ― 7.28 (m, 5H), 6.94 ― 6.90 (d, *J* = 6.8 Hz, 1 H), 5.51 (m, 1 H), 5.15 (s, 2H), 4.69 ― 4.64 (m, 1 H), 3.83 ― 3.81 (m, 2H), 1.62 ― 1.51 (m, 3H), 1.44(s, 9H), 0.91 ― 0.89(m, 6H); ¹³C NMR (100 MHz, CDCl₃) δ 172.6, 169.4, 156.0, 135.3, 128.5, 128.4, 128.2, 128.0, 80.0, 66.9, 50.7, 44.1, 41.1, 28.1, 24.7, 22.7, 21.7; ESI-MS calcd for [C₂₀H₃₀N₂O₅ + H⁺] 379.2, found: 379.3.

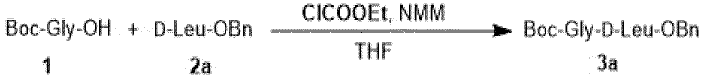

Or, as shown in the above formula, Boc-Gly-OH **1** (8.75 g, 50 mmol) was added into 100 mL of anhydrous THF, and stirred for 5 min under the protection of argon gas in an ice-salt bath, to lower the temperature of the reaction solution down to ― 15 °C. After adding NMM (6.2 mL, 55 mmol), ethyl chloroformate (5.3 mL, 55 mmol) was slowly added dropwise into the reaction solution, and stirred for 5 min. Then, the solution of D-Leu-OBn **2a** *p*-toluenesulphonate (19.7 g, 50 mmol) and NMM (6.2 mL) in anhydrous DMF was added, and stirred continuously at -15 °C to react for half hour. The reaction mixture was naturally warmed to room temperature with the ice-salt bath removed, and the reaction was continued overnight. After the reaction was completed, the insoluble was removed by suction filtration. The filtrate was concentrated under reduced pressure, and then dissolved in ethyl acetate. The ethyl acetate phase was washed successively with 1 M NaOH solution, water, 1 M hydrochloric acid solution and a small amount of saturated brine, dried with anhydrous sodium sulfate, and concentrated under reduced pressure. 17.30 g of Boc-Gly-D-Leu- OBn (**3a**), a light yellow oily liquid, was obtained with a yield of 91%. 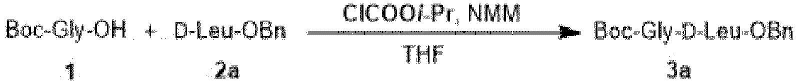 or, as seen in the above formula, Boc-Gly-OH **1** (8.75 g, 50 mmol) was added into 100 mL of anhydrous THF, and stirred for 5 min under the protection of argon gas in an ice-salt bath, to lower the temperature of the reaction solution down to ―15 °C. After adding NMM (6.2 mL, 55 mmol), **isopropyl chloroformate** (6.3 mL, 55 mmol) was slowly added dropwise into the reaction solution, and stirred for 5 min. Then, the solution of D-Leu-OBn **2a** *p*-toluenesulphonate (19.7 g, 50 mmol) and NMM (6.2 mL) in anhydrous DMF was added, and stirred continuously at -15 °C to react for 0.5 h. The reaction mixture was naturally warmed to room temperature with the ice-salt bath removed, and the reaction was continued overnight. After the reaction was completed, the insoluble was removed by suction filtration. The filtrate was concentrated under reduced pressure, and then dissolved in ethyl acetate. The ethyl acetate phase was washed successively with 1 M NaOH solution, water, 1 M hydrochloric acid solution and a small amount of saturated brine, dried with anhydrous sodium sulfate, and concentrated under reduced pressure. 17.67 g of Boc-Gly-D- Leu-OBn (**3a**), a light yellow oily liquid, was obtained with a yield of 93%.

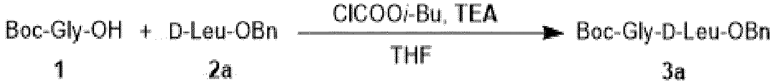

Or, as shown in the above formula, Boc-Gly-OH (8.75 g, 50 mmol) was added into 100 mL of anhydrous THF, and stirred for 5 min under the protection of argon gas in an ice-salt bath, to lower the temperature of the reaction solution down to -15 °C. After adding **TEA** (triethylamine, 7.6 mL, 55 mmol), isobutyl chloroformate (7 mL, 55 mmol) was slowly added dropwise into the reaction solution, and stirred for 5 min. Then, the solution of D-Leu-OBn p-toluenesulphonate (19.7 g, 50 mmol) and TEA (7.6 mL) in anhydrous DMF was added, and stirred continuously at ―15 °C to react for 0.5 h. The reaction mixture was naturally warmed to room temperature with the ice-salt bath removed, and the reaction was continued overnight. After the reaction was completed, the insoluble was removed by suction filtration. The filtrate was concentrated under reduced pressure, and then dissolved in ethyl acetate. The ethyl acetate phase was washed successively with 1 M NaOH solution, water, 1 M hydrochloric acid solution and a small amount of saturated brine, dried with anhydrous sodium sulfate, and concentrated under reduced pressure. 16.48 g of Boc-Gly-D- Leu-OBn (3a), a light yellow oily liquid, was obtained with a yield of 87%.

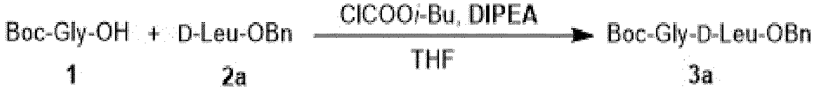

Or, as shown in the above formula, Boc-Gly-OH 1 (8.75 g, 50 mmol) was added into 100 mL of anhydrous THF, and stirred for 5 min under the protection of argon gas in an ice-salt bath, to lower the temperature of the reaction solution down to -15 °C. After adding **DIPEA** (diisopropylethylamine, 9.6 mL, 55 mmol), isobutyl chloroformate (7 mL, 55 mmol) was slowly added dropwise into the reaction solution, and stirred for 5 min. Then, the solution of D-Leu-OBn **2a** *p*-toluenesulphonate (19.7 g, 50 mmol) and DIPEA (9.6 mL) in anhydrous DMF was added, and stirred continuously at -15 °C to react for 0.5 h. The reaction mixture was naturally warmed to room temperature with the ice-salt bath removed, and the reaction was continued overnight. After the reaction was completed, the insoluble was removed by suction filtration. The filtrate was concentrated under reduced pressure, and then dissolved in ethyl acetate. The ethyl acetate phase was washed successively with 1 M NaOH solution, water, 1 M hydrochloric acid solution and a small amount of saturated brine, dried with anhydrous sodium sulfate, and concentrated under reduced pressure. 15.47 g of Boc-Gly-D-Leu-OBn (**3a**), light yellow oily liquid, was obtained with a yield of 81%.

1.2 Synthesis of Boc-Gly-D-Phe-OBn (3b)

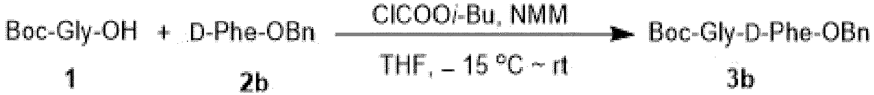

Boc-Gly-OH **1** (8.75 g, 50 mmol) was added into 100 mL of anhydrous THF, and stirred for 5 min under the protection of argon gas in an ice-salt bath, to lower the temperature of the reaction solution down to -15 °C. After adding NMM (6.2 mL, 55 mmol), isobutyl chloroformate was slowly added dropwise into the reaction solution (7 mL, 55 mmol), and stirred for 5 min. Then, the solution of D-Phe-OBn **2b** *p*-toluenesulphonate (21.35 g, 50 mmol) and NMM (6.2 mL) in anhydrous DMF was added, and stirred continuously at -15 °C to react for 0.5 h. The reaction mixture was naturally warmed to room temperature with the ice-salt bath removed, and the reaction was continued overnight. After the reaction was completed, the insoluble was removed by suction filtration. The filtrate was concentrated under reduced pressure, and then dissolved in ethyl acetate. The ethyl acetate phase was washed successively with 1 M NaOH solution, water, 1 M hydrochloric acid solution and a small amount of saturated brine, dried with anhydrous sodium sulfate, and concentrated under reduced pressure. A light yellow oily Boc-Gly-D-Phe-OBn (**3b**) was obtained and then purified by column chromatography with petroleum ether and ethyl acetate to obtain 19.16 g of a white solid, with a yield of 93%. White solid, Mp 73.5―75 °C; [α]_{D}²⁵= ― 11.6 (*c* 0.5, CHCl₃); ¹H NMR (600 MHz, CDCl₃) δ 7.35 (m, 3H), 7.30 ― 7.27 (m, 1H), 7.23 ― 7.18 (m, 2H), 7.13 ― 6.86 (m, 2H), 6.63 (d, *J =* 5.7 Hz, 1H), 5.16 ― 5.08 (m, 2H), 4.92 (dd, *J =* 6, 12 Hz, 1H), 3.86 ― 3.71 (m, 2H), 3.15 ― 3.05 (m, 2H), 1.44 (d, *J=* 8.5 Hz, 9H). ¹³C NMR (150MHz, CDCl₃) δ 171.1, 169.1, 135.5, 135.0, 129.3, 128.6, 128.6, 128.5, 128.5, 128.4, 128.2, 127.1, 77.2, 77.0, 76.8, 67.3, 53.1, 41.4, 37.9, 28.3(s), 24.8, 22.8, 21.8; ESI-MS calcd for [C₂₃H₂₈N₂O₅ + H⁺] 413.2, found 413.2.

### (II) Synthesis of Compound 4

### 2.1 Synthesis of NH₂-Gly-D-Leu-OBn (4a)

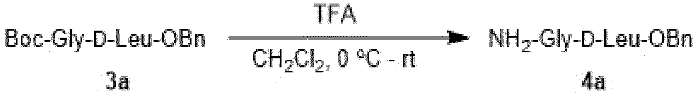

The dipeptide Boc-Gly-D-Leu-OBn **3a** (19 g) obtained from the above reaction was dissolved in 50 mL of dichloromethane. The reaction solution was cooled to 0 °C, and slowly added with **TFA** (trifluoroacetic acid, 50 mL) dropwise thereto. After returned to room temperature naturally and stirred for 5 h, the reaction solution was concentrated under reduced pressure to remove TFA. The residue was dissolved in dichloromethane, and adjusted to about pH = 9.0 with 1.0 M NaOH aqueous solution under the condition of 0 °C. The resulting organic phase was seperated out, and the water phase was extracted with dichloromethane (20 mL × 3). The organic phases were combined together and successively washed with a small amount of saturated brine, dried with anhydrous sodium sulfate, then concentrated under reduced pressure.

NH₂-Gly-D-Leu-OBn (**4a**), a colorless oily liquid, was obtain as the product with a yield of 13.25 g (95%). The product was used in the next reaction directly without purification.

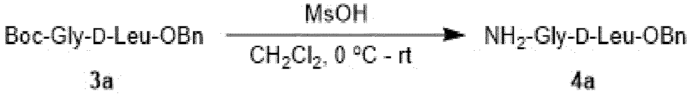

Or, as shown in the above formula, the dipeptide Boc-Gly-D-Leu-OBn **3a** (19 g) obtained from the above reaction was dissolved in 50 mL of dichloromethane. The reaction solution was cooled to 0 °C, and slowly added with **methanesulfonic acid** (50 mL) dropwise thereto. After returned to room temperature naturally and stirred for 5 h, the reaction solution was adjusted to about pH = 9.0 with 1.0 M NaOH aqueous solution under the condition of 0 °C. The resulting organic phase was seperated out, and the water phase was extracted with dichloromethane (20 mL × 3). The organic phases were combined together and successively washed with a small amount of saturated brine, dried with anhydrous sodium sulfate, then concentrated under reduced pressure.

NH₂-Gly-D-Leu-OBn (**4a**), a colorless oily liquid, was obtain as the product with a yield of 12.83 g (92%). The product was used in the next reaction directly without purification.

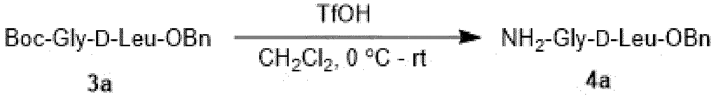

Or, as shown in the above formula, the dipeptide Boc-Gly-D-Leu-OBn **3a** (19 g) obtained from the above reaction was dissolved in 50 mL of dichloromethane. The reaction solution was cooled to 0 °C, and slowly added with **trifluoromethanesulfonic acid** (50 mL) dropwise thereto. After returned to room temperature naturally and stirred for 5 h, the reaction solution was adjusted to about pH = 9.0 with 1.0 M NaOH aqueous solution under the condition of 0 °C. The resulting organic phase was seperated out, and the water phase was extracted with dichloromethane (20 mL × 3). The organic phases were combined together and successively washed with a small amount of saturated brine, dried with anhydrous sodium sulfate, then concentrated under reduced pressure.

NH₂-Gly-D-Leu-OBn (**4a**), a colorless oily liquid, was obtain as the product with a yield of 12.7 g (91%). The product was used in the next reaction directly without purification

### 2.2 Synthesis of H₂N-Gly-D-Phe-OBn (4b)

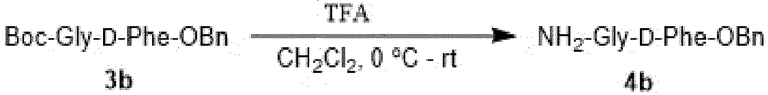

The dipeptide Boc-Gly-D-Phe-OBn **3b** (18.2 g, 44 mmol) obtained from the above reaction was dissolved in 44 mL of dichloromethane. The reaction solution was cooled to 0 °C, to which TFA (44 mL) was slowly added dropwise. After returned to room temperature naturally and stirred for 5 h, the reaction solution was concentrated under reduced pressure to remove away TFA. The residue was dissolved in dichloromethane, and adjusted to about pH = 9.0 with 1 M NaOH aqueous solution under the condition of 0°C. The resulting organic phase was seperated out, and the water phase was extracted with dichloromethane (20 mL × 3). The organic phases were combined together and successively washed with a small amount of saturated brine, dried with anhydrous sodium sulfate, and concentrated under reduced pressure.

NH₂-Gly-D-Leu-OBn (**4b**), a colorless oily liquid, was obtained as the product with a yield of 12.5 g (91%). The product was used in the next reaction directly without purification.

### (III) Synthesis of the intermediate compound 6

### 3.1 Synthesis of Boc-Pro-Gly-D-Leu-OBn (6a)

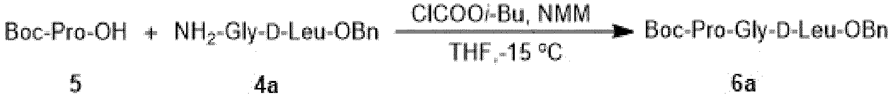

Boc-Pro-OH **5** (9.68 g, 45 mmol) was added into 100 mL of anhydrous THF, and stirred for 5 min under the protection of argon gas in an ice-salt bath to make the temperature of the reaction solution down to -15 °C. After adding NMM (5.6 mL, 50 mmol), isobutyl chloroformate (6.4 mL, 50 mmol) was slowly added dropwise into the reaction solution, and stirred for 5 min. Then, the solution of dipeptide NH₂-Gly-D-Leu-OBn **4a** (12.56 g, 45 mmol) and NMM (5.6 mL) in anhydrous THF was added. The reaction solution was stirred continuously for 30 min in the ice-salt bath, and then allowed to return to room temperature with the ice-salt bath removed away, and stirred to react until the completion of the reaction. The insolubles was removed by suction filtration, and the filtrate was concentrated under reduced pressure. The resulting oily residue was dissolved in ethyl acetate, and then successively washed with 1.0 M NaOH solution, water, 1.0 M hydrochloric acid solution and saturated brine, dried with anhydrous sodium sulfate, and concentrated under reduced pressure. Boc-Pro-Gly-D-Leu-OBn (**6a**), a light yellow oily liquid was obtained and purified by column chromatography with petroleum ether and ethyl acetate to obtain 20.33 g of product, with a yield of 95%. White solid, Mp: 104―105 °C; [α]_{D}²⁰ = ― 27 (c 1.0, CHCl₃); ¹H NMR (400 MHz, CDCl₃) δ 7.38 ― 7.32 (m, 5H), 7.22 (br, 1H), 7.03 (br, 1H), 5.08 ― 5.07 (dd, *J =* 12.8Hz, *J* = 21.6 Hz, 2H), 4.57 ― 4.55 (m, 1H), 4.25 ― 4.11 (m, 2H), 3.86 ― 3.81 (dd, *J =* 4.8 Hz, *J* = 17.2 Hz, 1H), 3.50 ― 3.40 (m, 2H), 2.15 ― 2.11 (m, 2H), 1.92 ― 1.89 (m, 2H), 1.60 ― 1.73(m, 3H), 1.44 (s, 9H), 0.92 ― 0.89 (m, 6H); ¹³C NMR (100 MHz, CDCl₃) δ 172.5, 169.3, 155.8, 128.5, 128.1, 128.0, 109.7, 80.8, 66.7, 60.9, 51.0, 47.4, 42.8, 40.1, 29.5, 28.3, 24.7, 24.6, 22.8, 21.5; ESI-MS calcd for [C₂₅H₃₇N₃O₆ + H⁺] 476.3, found: 476.3..

### 3.2 Synthesis of Boc-Pro-Gly-D-Phe-OBn (6b)

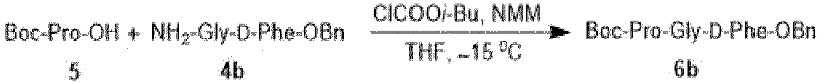

Boc-Pro-OH **5** (7.53 g, 35 mmol) was added into 100 mL of anhydrous THF, and stirred for 5 min under the protection of argon gas in an ice-salt bath to make the temperature of the reaction solution down to -15 °C. After adding NMM (4.3 mL, 38.5 mmol), isobutyl chloroformate (4.9 mL, 38.5 mmol) was slowly added dropwise into the reaction solution, and stirred for 5 min. Then, the solution of dipeptide NH₂-Gly-D-Phe-OBn **4b** (10.92 g, 35 mmol) and NMM (4.3 mL) in anhydrous THF was added. The reaction solution was stirred continuously for 30 min in the ice-salt bath, and then allowed to return to room temperature with the ice-salt bath removed away, and stirred to react until the completion of the reaction. The insolubles was removed by suction filtration, and the filtrate was concentrated under reduced pressure. The resulting oily residue was dissolved in ethyl acetate, and then successively washed with 1 M NaOH solution, water, 1 M hydrochloric acid solution and saturated brine, dried with anhydrous sodium sulfate, concentrated under reduced pressure, and purified by column chromatography with petroleum ether and ethyl acetate. 17.1 g of Boc-Pro-Gly-D-Phe-OBn (**6b**) was obtained as the product with a yield of 96%. White solid, Mp 100-102 °C; [α]_{D}²⁵ = ― 66.0 (c 0.5, CHCl₃); ¹H NMR (600 MHz, CDCl₃) δ 7.34 (d, *J=* 6.5 Hz, 2H), 7.27 (s, 1H), 7.21 (t, *J* = 7.3 Hz, 2H), 7.13 ― 6.89 (m, 3H), 6.54 (m, 1H), 5.12 (t, *J=* 14.8 Hz, 2H), 4.84 (s, 1H), 4.22 (s, 1H), 3.92 (d, *J=* 33.6 Hz, 2H), 3.51 ― 3.33 (m, 2H), 3.17 ― 3.05 (m, 2H), 2.23 ― 2.00 (m, 2H), 1.86 (s, 2H), 1.45 (s, 9H). ¹³C NMR (150 MHz, CDCl₃) δ 172.5, 171.1, 168.8, 136.1, 135.3, 129.3,129.2, 128.6,128.5,128.4, 127.0, 80.7, 77.3, 77.0, 76.8, 67.1, 60.6, 53.6, 47.32, 43.0, 37.6, 28.4, 24.6; ESI-MS calcd for [C₂₈H₃₅N₃O₆ + H⁺] 510.2, found 510.2.

### (IV) Synthesis of the intermediate compound 7

### 4.1 Synthesis of NH₂-Pro-Gly-D-Leu-OBn (7a)

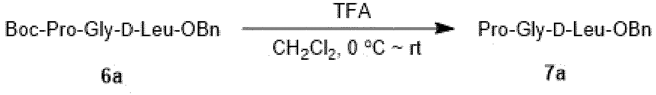

The synthetized tripeptide Boc-Pro-Gly-D-Leu-OBn **6a** (19 g, 40 mmol) was dissolved completely in CH₂Cl₂ (40 mL), and slowly added with TFA (40 mL) dropwise at 0 °C. After returned to room temperature, the reaction solution was stirred for 5 h. The resulting reaction solution was concentrated under reduced pressure to remove TFA as much as possible. The residue was dissolved in CH₂Cl₂, and then neutralized with 1 M NaOH solution at 0 °C to about pH = 9.0. The organic phase was seperated out, and the water phase was extracted with CH₂Cl₂ (20 mL ×3). The organic phases were combined, washed with saturated brine once, dried with anhydrous sodium sulfate, and concentrated under reduced pressure. 13.54 g of NH₂-Pro-Gly-D-Leu-OBn (**7a**), a colorless oily liquid, was obtained with a yield of 90%. The product was used in the next reaction directly without purification.

### 4.2 Synthesis of Pro-Gly-D-Phe-OBn (7b)

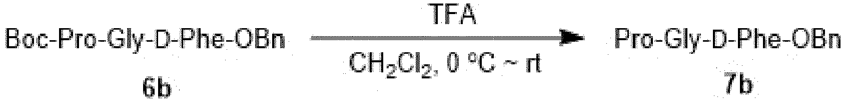

The synthetized tripeptide Boc-Pro-Gly-D-Phe-OBn **6b** (16.5 g, 32 mmol) was dissolved completely in CH₂Cl₂ (32 mL), and slowly added with TFA (32 mL) dropwise at 0 °C. After returned to room temperature, the reaction solution was stirred for 5 h. The resulting reaction solution was concentrated under reduced pressure to remove TFA as much as possible. The residue was dissolved in CH₂Cl₂, and then neutralized with 1 M NaOH solution at 0 °C to about pH = 9.0. The organic phase was seperated out, and the water phase was extracted with CH₂Cl₂ (20 mL ×3). The organic phases were combined, washed with saturated brine once, dried with anhydrous sodium sulfate, and concentrated under reduced pressure. 11.8 g of Pro-Gly-D-Phe-OBn (**7**b), a colorless oily liquid, was obtained with a yield of 90%. The product was used in the next reaction directly without purification

### (V) Synthesis of the intermediate compound 9

### 5.1 Synthesis of Boc-D-Val-Pro-Gly-D-Leu-OBn (9a)

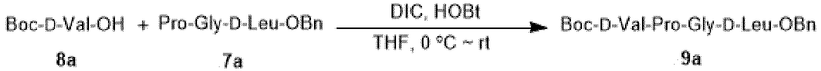

To a 150 mL round-bottomed flask, HOBt (4.45 g, 33 mmol) and Boc-D-Val-OH **8a** (6.45 g, 30 mmol) were added, and 60 mL of dried THF was added under the protection of argon gas and stirred at 0 °C. When the temperature inside the flask decreased to 0 °C, **DIC (N,N'-diisopropylcarbodiimide**, 5.1 mL, 33 mmol) was slowly added dropwise. After 0.5 h, the tripeptide Pro-Gly-D-Leu-OBn **7a** (11.28 g, 30 mmol) in THF was added. After stirring for 48 h at room temperature, the insoluble was removed away by filtration, and the filtrate was concentrated to dryness under reduced pressure. The residue was dissolved in ethyl acetate, and then successively washed with 1 M NaOH solution, water, 1 M hydrochloric acid solution and saturated brine, dried with anhydrous sodium sulfate, concentrated under reduced pressure, and then purified by petroleum ether and ethyl acetate column chromatography. 14.84 g of tetrapeptide Boc-D-Val- Pro-Gly-D-Leu-OBn (**9a**), a white solid, was obtained with a yield of 86%. White solid, Mp: 123.5―125.5 °C; [α]_{D}²⁰ = + 15 (c 0.7, CHCl₃); ¹H NMR (400 MHz, CDCl₃) δ 7.36 ― 7.30 (m, 5H), 7.24 ― 7.23 (m, 1 H), 5.34 ― 5.32 (d, *J* = 6.8 Hz, 1 H), 5.20 ― 5.13 (dd, *J =* 12.4 Hz, *J =* 14.8 Hz, 2H), 4.64 ― 4.59 (m, 1H), 4.50 ― 4.50 (t, *J =* 6.0 Hz, l H), 4.20 ― 4.14 (dd, *J* = 7.2 *Hz, J* = 16.8 Hz, 1 H), 4.07 (m, 1 H), 3.98 ― 3.94 (m, 1 H), 3.65 ― 3.56 (m, 2H), 2.21 ― 2.16 (m, 2H), 2.09 ― 1.99 (m, 2H), 1.95 ― 1.90 (m, 1 H), 1.76 ― 1.61 (m, 3H), 1.40 (s, 9H), 1.01 ― 1.00 (d, *J=* 6.8 Hz, 3H), 0.97 ― 0.95 (d, *J* = 6.8 Hz, 3H), 0.92 ― 0.88 (m, 6H); ¹³C NMR (100 MHz, CDCl₃) δ 172.6, 171.7, 169.0, 156.9,135.7, 128.4, 128.0, 127.8, 80.3, 66.4, 61.0, 58.2, 50.7, 47.6, 43.1, 40.7, 30.1, 29.2, 28.1, 24.6, 23.4, 22.8, 21.7, 19.1, 18.8; ESI-MS calcd for [C₃₀H₄₆N₄O₇+H⁺] 575.3, found 575.3.

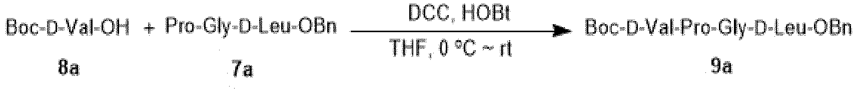

Or, as shown in the above formula, to a 150 mL round-bottomed flask, HOBt (4.45 g, 33 mmol) and Boc-D-Val-OH **8a** (6.51 g, 30 mmol) were added, and 60 mL of dried THF was added under the protection of argon gas and stirred at 0 °C. When the temperature inside the flask decreased to 0°C, a solution of **DCC (N,N'-dicyclohexylcarbodiimide**, 6.8 g, 33 mmol) in DMF was slowly added dropwise. After 0.5 h, the tripeptide Pro-Gly-D-Leu-OBn **7a** (11.28 g, 30 mmol) in THF was added. After stirring for 48 h at room temperature, the insoluble was removed away by filtration, and the filtrate was concentrated to dryness under reduced pressure. The residue was dissolved in ethyl acetate, and then successively washed with 1 M NaOH solution, water, 1 M hydrochloric acid solution and saturated brine, dried with anhydrous sodium sulfate, concentrated under reduced pressure and then purified by column chromatography with petroleum ether and ethyl acetate. 12.42 g of tetrapeptide Boc-D-Val-Pro-Gly-D-Leu-OBn (**9**a), a white solid, was obtained with a yield of 72%.

### 5.2 Synthesis of Boc-D-Tle-Pro-Gly-D-Leu-OBn (9b)

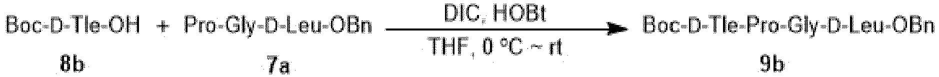

To a 150 mL round-bottomed flask, HOBt (4.45 g, 33 mmol) and Boc-D-Tle-OH **8b** (6.93 g, 30 mmol) were added, and 60 mL of dried THF was added under the protection of argon gas and stirred at 0 °C. When the temperature inside the flask decreased to 0 °C, DIC (5.1 mL, 33 mmol) was added slowly dropwise. After 0.5 h, a solution of the tripeptide Pro-Gly-D-Leu-OBn **7a** (11.28 g, 30 mmol) in THF was added. After stirring for 48 h at room temperature, the insoluble was removed away by filtration, and the filtrate was concentrated to dryness under reduced pressure. The residue was dissolved in ethyl acetate, and then successively washed with 1 M NaOH solution, water, 1 M hydrochloric acid solution and saturated brine, dried with anhydrous sodium sulfate, concentrated under reduced pressure and then purified by petroleum ether and ethyl acetate column chromatography. 15.02 g of tetrapeptide Boc-D-Tle-Pro-Gly-D-Leu-OBn (**9b**), a white solid, was obtained with a yield of 85%. White solid, Mp 168―169.4 °C; [α]_{D}²⁵ = + 9.0 (c 0.5, CHCl₃); ¹H NMR (600 MHz, CDCl₃) δ 7.47 (d, *J=* 7.7 Hz, 1H), 7.35 (d, *J=* 4.4 Hz, 3H), 7.33 ― 7.28 (m, 1H), 7.08 ― 7.03 (m, 1H), 5.48 (d, *J* = 7.1 Hz, 1H), 5.19 ― 5.12 (m, 2H), 4.62 ― 4.55 (m, 1H), 4.46 (dd, *J* = 7.5, 4.9 Hz, 1H), 4.22 (dd, *J* = 17.2, 7.9 Hz, 1H), 4.15 ― 4.10 (m, 1H), 4.07 (d, *J* = 7.1 Hz, 1H), 3.65 ― 3.59 (m, 1H), 3.56 (dd, *J =* 17.1, 5.1 Hz, 1H), 2.20 ― 2.15 (m, 2H), 2.09 ― 2.03 (m, 1H), 2.00 (dd, *J* = 11.3, 5.3 Hz, 1H), 1.77 ― 1.56 (m, 4H), 1.40 (s, 9H), 1.04 (s, 9H), 0.88 (dd, *J* = 12.8, 6.4 Hz, 6H). ¹³C NMR (150 MHz, CDCl₃) δ 172.7, 172.2, 171.7, 169.1, 135.91, 128.5, 128.1,128.0, 77.24, 77.0, 76.8, 66.4, 61.3, 59.7, 50.9, 48.3, 43.2, 40.7, 33.9, 29.4, 28.2, 26.5, 24.8, 24.7, 22.8, 21.8; ESI-MS calcd for [C₃₁H₄₈N₄O₇ + H⁺] 588.7, found 588.7.

### 5.3 Synthesis of Boc-D-Phg-Pro-Gly-D-Leu-OBn (9c)

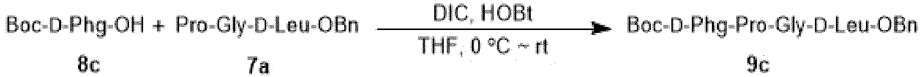

To a 150 mL round-bottomed flask, HOBt (4.45 g, 33 mmol) and Boc-D-Phg-OH **8c** (7.53 g, 30 mmol) were added, and 60 mL of dried THF was added under the protection of argon gas and stirred at 0°C. When the temperature inside the flask decreased to 0 °C, DIC (5.1 mL, 33 mmol) was slowly added dropwise. After 0.5 h, a solution of the tripeptide Pro-Gly-D-Leu-OBn **7a** (11.28 g, 30 mmol) in THF was added. After stirring for 48 h at room temperature, the insoluble was removed away by filtration, and the filtrate was concentrated to dryness under reduced pressure. The residue was dissolved in ethyl acetate, and then successively washed with 1 M NaOH solution, water, 1 M hydrochloric acid solution and saturated brine, dried with anhydrous sodium sulfate, and concentrated under reduced pressure. 15.52 g of tetrapeptide Boc-D-Phg-Pro-Gly-D-Leu-OBn (**9c**) was obtained with a yield of 85%. White solid, Mp 142―144°C; [α]_{D}²⁵ = ― 2.0 (c 0.5, CHCl₃); ¹H NMR (600 MHz, CDCl₃) δ 7.45 ― 7.39 (m, 2H), 7.38 ― 7.32 (m, 6H), 7.27 (s, 1H), 7.22 (d, *J =* 7.0 Hz, 1H), 5.72 (d, *J* =5.2 Hz, 2H), 5.38-5.30 (m, 1H), 5.17 (d, *J =* 3.3 Hz, 2H), 4.66 ― 4.61 (m, 1H), 4.47 ― 4.39 (m, 1H), 4.04 (dd, *J =* 16.8, 6.6 Hz, 1H), 3.98― 3.94 (m, 1H), 3.83 (dd, *J* = 12.7, 6.3 Hz, 1H), 3.38 ― 3.29 (m, 1H), 2.21 ― 2.16 (m, 1H), 2.09― 1.99 (m, 2H), 1.75 ― 1.70 (m, 2H), 1.39 (d, *J* = 4.9 Hz, 9H), 1.13 (d, *J* = 6.5 Hz, 4H), 0.96 ― 0.90 (m, 6H). ¹³C NMR (150 MHz, CDCl₃) δ 172.5, 171.5, 169.0, 135.7, 129.2, 129.1, 128.5, 128.4, 128.1 (dd, *J* = 12.3, 9.2 Hz), 80.5, 77.2, 77.0, 76.8, 66.8, 61.2, 51.0, 47.3, 43.4, 42.1, 41.1, 28.2, 24.6, 23.4, 22.8, 21.8; ESI-MS calcd for [C₃₃H₄₄N₄O₇ + H⁺] 609.3, found 609.3.

### 5.4 Synthesis of the tetrapeptide Boc-D-Chg-Phe-Pro-Gly-D-Leu-OBn (9d)

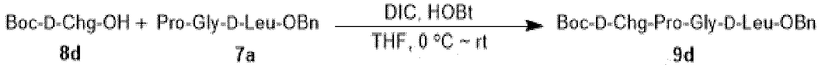

To a 150 mL round-bottomed flask, HOBt (4.45 g, 33 mmol), Boc-D-Chg-OH **8d** (7.71 g, 30 mmol) were added, and 60 mL of dried THF was added under the protection of argon gas and stirred at 0 °C. When the temperature inside the flask decreased to 0 °C, DIC (5.1 mL, 33 mmol) was slowly added dropwise. After 0.5 h, a solution of the tripeptide Pro-Gly-D-Leu-OBn **7a** (11.28 g, 30 mmol) in THF was added. After stirring for 48 h at room temperature, the insoluble was removed away by filtration, and the filtrate was concentrated to dryness under reduced pressure. The residue was dissolved in ethyl acetate, and then successively washed with 1 M NaOH solution, water, 1 M hydrochloric acid solution and saturated brine, dried with anhydrous sodium sulfate, concentrated under reduced pressure, and purified by column chromatography with petroleum ether and ethyl acetate. 15.5 g of tetrapeptide Boc-D-Chg-Phe-Pro-Gly-D-Leu-OBn (**9**d) was obtained with a yield of 84%. White solid, Mp: 160―161 °C; [α]_{D}²⁰= ― 10 (c 0.62, CHCl₃); ¹H NMR (400 MHz, CDCl₃) δ 7.37 ― 7.30 (m, 5H), 5.28 ― 5.26 *(d, J =* 6.4 Hz, 1 H), 5.21 ― 5.13 (m, 2H), 4.66 ― 4.60 (m, 1H), 4.50 ― 4.47 (m, 1H), 4.20 ― 4.07 (m, 2H), 4.00 ― 3.96 (m, 1H), 3.64 ― 3.56 (m, 2H), 2.21 ― 2.16 (m, 2H), 2.10 ― 1.98 (m, 2H), 1.87 ― 1.84 (m, 1H), 1.79 ― 1.56 (m, 8H), 1.39 (s, 9H), 1.28 ― 1.17 (m, 4H), 1.31 ― 1.11 (d, *J* = 6.8 Hz, 2H), 1.06 ― 0.99 (m, 2H), 0.92 ― 0.89 (m, 6H); ¹³C NMR (100 MHz, CDCl₃) δ 172.5, 171.7, 169.0, 157.0, 135.7, 128.4, 128.0, 127.8, 80.3, 66.3, 60.9, 57.6, 50.7, 47.6, 43.1, 41.8, 40.8, 39.5, 29.4, 29.2, 28.1, 26.0, 25.7, 25.6, 24.6, 23.4, 22.8, 21.7; ESI-MS calcd for [C₃₃H₅₀N₄O₇ + H⁺] 614.4, found: 614.4.

### 5.5 Synthesis of Boc-D-Phe-Pro-Gly-D-Leu-OBn (9e)

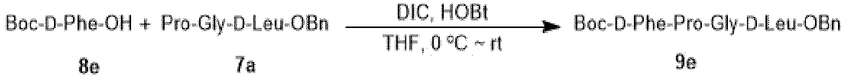

To a 150 mL round-bottomed flask, HOBt (4.45 g, 33 mmol) and Boc-D-Phe-OH **8e** (7.95 g, 30 mmol) were added, and 60 mL of dried THF was added under the protection of argon gas and stirred at 0 °C. When the temperature inside the flask decreased to 0 °C, DIC (5.1 mL, 33 mmol) was slowly added dropwise. After 0.5 h, a solution of the tripeptide Pro-Gly-D-Leu-OBn **7a** (11.28 g, 30 mmol) in THF was added. After stirring for 48 h at room temperature, the insoluble was removed away by filtration, and the filtrate was concentrated to dryness under reduced pressure. The residue was dissolved in ethyl acetate, and then successively washed with 1 M NaOH solution, water, 1 M hydrochloric acid solution and saturated brine, dried with anhydrous sodium sulfate, concentrated under reduced pressure, and purified by column chromatography with petroleum ether and ethyl acetate. 16.07 g of tetrapeptide Boc-D-Phe-Pro-Gly-D-Leu-OBn (**9e**) was obtain with a yield of 86%. White solid, Mp:160―161 °C; [α]_{D}²⁰ = ― 10 (c 0.62, CHCl₃); ¹H NMR (400 MHz, CDCl₃) δ 7.40 ― 7.19 (m, 10H), 5.50 (br, 1H), 5.18 (s, 1H), 4.65 ― 4.64 (m, 1 H), 4.41 ― 4.38 (m, 1H), 4.32 ― 4.30 (m, 1H), 4.16 ― 4.10 (m, 1H), 3.69 ― 3.61 (m, 2H), 2.68 ― 2.65 (m, 1H), 2.05 ― 2.02 (m, 1H), 1.91 ― 1.56 (m, 8H), 1.39 (s, 9H), 1.14 ― 1.12 (m, 2H), 0.96 ― 0.91 (m, 6H); ¹³C NMR (100 MHz, CDCl₃) δ 172.6, 172.0, 171.5, 169.0 156.3,135.9, 135.7, 129.2, 128.6, 128.5, 128.1, 127.9, 127.2, 80.5, 66.5, 60.9, 54.4, 50.7, 47.1, 43.1, 42.0, 40.9, 3 8.1, 28.8, 28.1, 24.6, 24.4, 23.4, 22.9, 21.7; ESI-MS calcd for [C₃₄H₄₆N₄O₇+H⁺] 623.3, found 623.3..

### 5.6 Synthesis of Boc-D-Phe-Pro-Gly-D-Phe-OBn (9f)

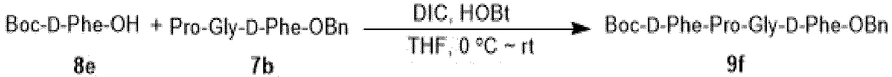

To a 150 mL round-bottomed flask, HOBt (3.71g, 27.5 mmol) and Boc-D-Phe-OH **8e** (6.63, 25 mmol) were added, and 50 mL of dried THF was added under the protection of argon gas and stirred at 0 °C. When the temperature inside the flask decreased to 0 °C, DIC (4.25 mL, 27.5 mmol) was slowly added dropwise. After 0.5 h, a solution of the tripeptide Pro-Gly-D-Phe-OBn **7b** (10.23 g, 25 mmol) in THF was added. After stirring for 48 h at room temperature, the insoluble was removed away by filtration, and the filtrate was concentrated to dryness under reduced pressure. The residue was dissolved in ethyl acetate, and then successively washed with 1 M NaOH solution, water, 1 M hydrochloric acid solution and saturated brine, dried with anhydrous sodium sulfate, concentrated under reduced pressure, and then purified by column chromatography with petroleum ether and ethyl acetate. 13.45 g of tetrapeptide Boc-D-Phe-Pro-Gly-D-Phe-OBn (**9f**) was obtained with a yield of 82%. White solid, Mp 138 ― 139.6 °C; [α]_{D}²⁵ = ― 49.0 (c 0.5, CHCl₃); ¹H NMR (600 MHz, CDCl₃) δ 7.33 ― 7.28 (m, 4H), 7.25 ― 7.18 (m, 8H), 7.16 ― 7.13 (m, 3H), 5.45 (d, *J=* 6.5 Hz, 1H), 5.13 ― 5.08 (m, 2H), 4.88 (dd, *J =* 14.4, 6.6 Hz, 1H), 4.46 ― 4.41 (m, 1H), 4.33 (dd, *J =* 7.8, 3.7 Hz, 1H), 3.98 (dd, *J* = 17.0, 6.9 Hz, 1H), 3.73 (dd, *J* = 17.0, 5.7 Hz, 1H), 3.66 ― 3.585 (b, 1H), 3.15 (dd, *J=* 6.7, 2.6 Hz, 2H), 2.95 (t, *J=* 7.7 Hz, 2H), 2.67 (dd, *J=* 16.8, 7.6 Hz, 1H), 2.08 ― 2.02 (m, 1H), 1.84 ― 1.75 (b, 3H), 1.57 ― 1.51 (b, 1H), 1.39 (s, 9H). ¹³C NMR (150 MHz, CDCl₃) δ 171.9, 171.5, 171.4, 168.8, 136.2, 135.4, 129.4, 129.3, 128.6, 128.5, 128.4, 128.3, 128.2, 127.2, 126.8, 80.5, 77.2, 77.0, 76.8, 66.9, 60.8, 53.6, 47.1, 43.2, 38.2, 28.7, 28.3, 24.4; ESI-MS calcd for [C₃₇H₄₄N₄O₇ + H⁺] 657.3, found 657.3.

### (VI) Synthesis of the intermediate compound 10

### 6.1 Synthesis of H₂N-D-Val-Pro-Gly-D-Leu-OBn (10a)

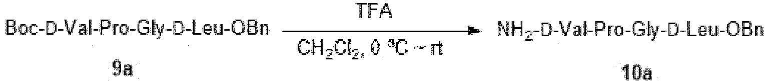

The tetrapeptide Boc-D-Val-Pro-Gly-D-Leu-OBn 9a (12.65 g, 22 mmol) synthetized above was dissolved in CH₂Cl₂ (20 mL), and then slowly added with TFA (20 mL) dropwise at 0 °C, and stirred at room temperature for 5 h. The resulting reaction solution was concentrated under reduced pressure to remove TFA as much as possible. The residue was dissolved in CH₂Cl₂, and then neutralized with 1 M NaOH solution at 0 °C to about pH = 9.0. The organic phase was seperated out, and the water phase was extracted with CH₂Cl₂ (20 mL ×3). The organic phases were combined, washed with saturated brine once, dried with anhydrous sodium sulfate, and concentrated under reduced pressure. 10.03 g of H₂N-D-Val-Pro-Gly-D-Leu-OBn (**10a**), a colorless oily liquid, was obtained with a yield of 96%. The product was used in the next reaction directly without purification.

### 6.2 Synthesis of H₂N-D-Tle-Pro-Gly-D-Leu-OBn (10b)

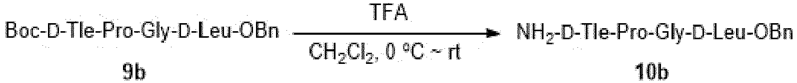

The tetrapeptide Boc-D-Tle-Pro-Gly-D-Leu-OBn **9b** (12.96 g, 22 mmol) synthetized above was dissolved in CH₂Cl₂ (25 mL), and then slowly added with TFA (25 mL) dropwise at 0 °C and stirred at room temperature for 5 h. The resulting reaction solution was concentrated under reduced pressure to remove TFA as much as possible. The residue was dissolved in CH₂Cl₂, and then neutralized with 1 M NaOH solution at 0 °C to about pH = 9.0. The organic phase was seperated out, and the water phase was extracted with CH₂Cl₂ (20 mL ×3). The organic phases were combined, washed with saturated brine once, dried with anhydrous sodium sulfate, and concentrated under reduced pressure. 10.22 g of H₂N-D-Tle-Pro-Gly-D-Leu-OBn (**10b**), a colorless oily liquid, was obtained with a yield of 95%. The product was used in the next reaction directly without purification.

### 6.3 Synthesis of H₂N-D-Phg-Pro-Gly-D-Leu-OBn (10c)

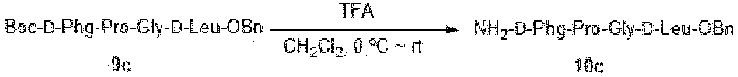

The tetrapeptide Boc-D-Phg-Pro-Gly-D-Leu-OBn **9c** (13.4 g, 22 mmol) synthetized above was dissolved in CH₂Cl₂ (25 mL), and then slowly added with TFA (25 mL) dropwise at 0 °C, and stirred at room temperature for 5 h. The resulting reaction solution was concentrated under reduced pressure to remove TFA as much as possible. The residue was dissolved in CH₂Cl₂, and then neutralized with 1 M NaOH solution at 0 °C to about pH = 9.0. The organic phase was seperated out, and the water phase was extracted with CH₂Cl₂ (20 mL ×3). The organic phases were combined, washed with saturated brine once, dried with anhydrous sodium sulfate, and concentrated under reduced pressure. 10.3 g of H₂N-D-Phg-Pro-Gly-D-Leu-OBn (**10c**), a colorless oily liquid, was obtained with a yield of 92%. The product was used in the next reaction directly without purification.

### 6.4 Synthesis of the tetrapeptide H₂N-D-Chg -Pro-Gly-D-Leu-OBn (10d)

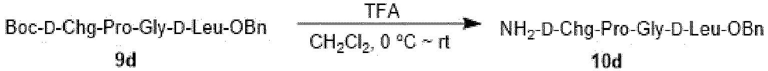

The tetrapeptide Boc-D-Chg-Pro-Gly-D-Leu-OBn **9d** (13.53 g, 22 mmol) synthetized above was dissolved in CH₂Cl₂ (25 mL), and then slowly added with TFA (25 mL) dropwise at 0 °C, and stirred at room temperature for 5 h. The resulting reaction solution was concentrated under reduced pressure to remove TFA as much as possible. The residue was dissolved in CH₂Cl₂, and then neutralized with 1 M NaOH solution at 0 °C to about pH = 11.0. The organic phase was seperated out, and the water phase was extracted with CH₂Cl₂ (20 mL ×3). The organic phases were combined, washed with saturated brine, dried with anhydrous sodium sulfate, and concentrated under reduced pressure sequentially. 10.6 g of H₂N-D-Chg-Pro-Gly-D-Leu-OBn (**10d**), a colorless oily liquid, was obtained with a yield of 94%. The product was used in the next reaction directly without purification

### 6.5 Synthesis of H₂N-D-Phe-Pro-Gly-D-Leu-OBn (10e)

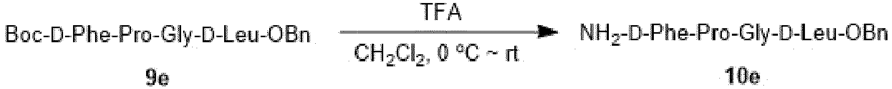

The tetrapeptide Boc-D-Phe-Pro-Gly-D-Leu-OBn 9e (13.7 g, 22 mmol) synthetized above was dissolved in CH₂Cl₂ (25 mL), and then slowly added with TFA (25 mL) dropwise at 0 °C, and stirred at room temperature for 5 h. The resulting reaction solution was concentrated under reduced pressure to remove TFA as much as possible. The residue was dissolved in CH₂Cl₂, and then neutralized with 1 M NaOH solution at 0 °C to about pH = 9.0. The organic phase was seperated out, and the water phase was extracted with CH₂Cl₂ (20 mL ×3). The organic phases were combined, washed with saturated brine, dried with anhydrous sodium sulfate, and concentrated under reduced pressure. 10.5 g of H₂N-D-Phe-Pro-Gly-D-Leu-OBn (**10e**), a colorless oily liquid, was obtained with a yield of 91%. The product was used in the next reaction directly without purification.

### 6.6 Synthesis of H₂N-D-Phe-Pro-Gly-D-Phe-OBn (10f)

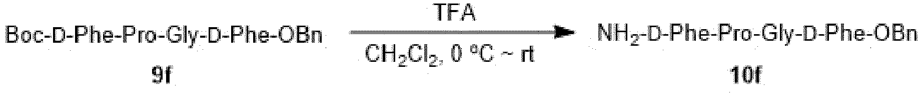

The tetrapeptide Boc-D-Phe-Pro-Gly-D-Phe-OBn **9f** (13.12 g, 20 mmol) synthetized above was dissolved in CH₂Cl₂ (25 mL), and then slowly added with TFA (25 mL) dropwise at 0 °C, and stirred at room temperature for 5 h. The resulting reaction solution was concentrated under reduced pressure to remove TFA as much as possible. The residue was dissolved in CH₂Cl₂, and then neutralized with 1 M NaOH solution at 0 °C to about pH = 11.0. The organic phase was seperated out, and the water phase was extracted with CH₂Cl₂ (20 mL ×3). The organic phases were combined, washed with saturated brine once, dried with anhydrous sodium sulfate, and concentrated under reduced pressure. 10.62 g of H₂N-D-Phe-Pro-Gly-D-Phe-OBn (**10f**), a colorless oily liquid was obtained with a yield of 94%. The product was used in the next reaction directly without purification.

### Example 2

The preparation methods of the sepecific compounds TP-1 to TP-6 of the present disclosure are given below.

### Synthesis of the tetrapeptide TP-1: NH₂-D-Val-Pro-Gly-D-Leu-OH

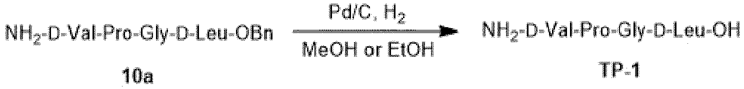

The NH₂-D-Val-Pro-Gly-D-Leu-OBn **10a** (10.0 g, 21 mmol) synthetized above was dissolved in **methanol** (20 mL), transferred into a 100 mL autoclave, and then added with 500 mg of Pd/C (5%), and stirred for 6 hours with hydrogen having been introduced to 8 Mpa at room temperature. Pd/C was removed away by filtration, and the filtrate was concentrated under reduced pressure. 8.0 g of tetrapeptide NH₂-D-Val-Pro-Gly-D-Leu-OH (**TP-**1) was obtained as the product with a yield of 100%. White solid, Mp: 152―154 °C; [α]_{D}²⁰ = ― 58.0 (c 0.85, H₂O); ¹H NMR (600 MHz, D₂O) 84.55 ― 4.50 (m, 1H), 4.31 ― 4.25 (m, 1H), 4.04 (d, *J* = 17.1 Hz, 1H), 3.90 (d, *J =* 17.1 Hz, 1H), 3.87 ― 3.83 (m, 1H), 3.77 ― 3.73 (m, 1H), 2.41 ― 2.33 (m, 2H), 2.11-2.04 (m, 3H), 1.74 ― 1.60 (m, 4H), 1.13 (d, *J=* 7.2 Hz ,3H), 1.05 (d, *J=* 12 Hz ,3H), 0.95 (d, *J =* 6 Hz 3H), 0.91 (d, *J =* 6 Hz,3H). ¹³C NMR (150 MHz, D₂O) δ 179.7, 174.3, 170.4, 168.9, 61.1, 57.1, 53.5, 48.1, 42.4, 40.5, 29.2, 28.7, 24.5, 24.3, 22.4, 20.5, 17.9, 15.9; HRMS calcd for [C₁₈H₃₂N₄O₅ + H⁺] 385.2445, found 385.2438.

The NH₂-D-Val-Pro-Gly-D-Leu-OBn **10a** (10.0 g) synthetized above was dissolved in **ethanol** (20 mL), transferred into a 100 mL autoclave, and then added with 500 mg of Pd/C (5%), and stirred for 6 hours with hydrogen having been introduced to 8 Mpa at room temperature. Pd/C was removed away by filtration, and the filtrate was concentrated under reduced pressure. 7.45 g of tetrapeptide NH₂-D-Val-Pro-Gly-D-Leu-OH (**TP**-1), white solid, was obtained as the product with a yeild of 93%.

### Example 2

### Synthesis of TP-2: H₂N-D-Tle-Pro-Gly-D-Leu-OH

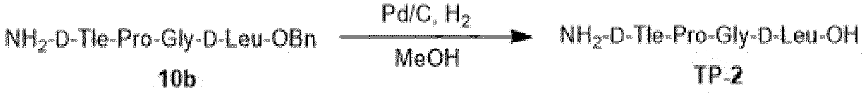

The NH₂-D-Tle-Pro-Gly-D-Leu-OBn **10b** (9.76 g, 20 mmol) synthetized above was dissolved in methanol (20 mL), transferred into a 100 mL autoclave, and then added with 500 mg of Pd/C (5%), and stirred for 6 hours with hydrogen having been introduced to 8 Mpa at room temperature. Pd/C was removed away by filtration, and the filtrate was concentrated under reduced pressure. 7.8 g of tetrapeptide NH₂-D-Tle-Pro-Gly-D-Leu-OH (**TP-2**), was obtained as the product with a yeild of 98%. White solid, Mp:204―205 °C, [α]_{D}²⁰= ― 28.0 (c 1.0, DMF); ¹H NMR (600 MHz, D₂O) 4.38 ― 4.34 (m, 1H), 4.12 ― 4.02 (m, 2H), 3.81 (q, *J=* 17.1 Hz, 2H), 3.72 ― 3.63 (m, 2H), 3.21 (d, *J =* 0.4 Hz, 1H), 2.24 ― 2.18 (m, 1H), 1.91 (s, 3H), 1.57 ― 1.52 (m, 1H), 1.50 ― 1.42 (m, 2H), 0.97 (s, 9H), 0.76 (dd, *J =* 22.8, 5.8 Hz, 6H); ¹³C NMR (150 MHz, D₂O) δ 179.8, 174.3, 170.4, 168.2, 61.0, 59.1, 53.7, 49.0, 42.4, 40.6, 33.9, 29.3, 25.3, 24.5, 24.26, 22.4, 20.5; HRMS calcd for [C₁₉H₃₀N₄O₅ + H⁺] 399.2602, found 399.2599.

### Example 3

### Synthesis of TP-3: H₂N-D-Phg-Pro-Gly-D-Leu-OH

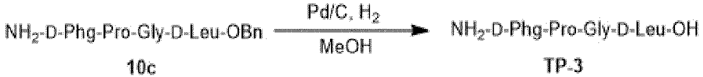

The NH₂-D-Phg-Pro-Gly-D-Leu-OBn **10c** (10.0 g, 19.5 mmol) synthetized above was dissolved in methanol (20 mL), transferred into a 100 mL autoclave, and then added with 500 mg of Pd/C (5%), and stirred for 6 hours with hydrogen having been introduced to 8 Mpa at room temperature. Pd/C was removed by filtration, and the filtrate was concentrated under reduced pressure. 7.8 g of tetrapeptide NH₂-D-Phg-Pro-Gly-D-Leu-OH (**TP-**3) was obtained as the product with a yield of 96%. White solid, Mp: 150 ― 153 °C, [α]_{D}²⁰= ― 94.0 (c 1.0, H₂O); ¹H NMR (600 MHz, D₂O) δ 7.62 ― 7.42 (m, 5H), 5.47 (s, 1H), 4.50 (dd, *J* = 9.0, 3.8 Hz, 1H), 4.31 (dd, *J* = 10.6, 3.2 Hz, 1H), 4.09 (d, *J* = 17.2 Hz, 1H), 3.89 *(d, J =* 17.2 Hz, 1H), 3.80 ― 3.71 (m, 1H), 3.07 ― 3.00 (m, 1H), 2.25 ― 2.15 (m, 1H), 2.05 ― 1.94 (m, 2H), 1.86 ― 1.72 (m, 2H), 1.69 ― 1.53 (m, 2H), 1.01 ― 0.86 (m, 6H); ¹³C NMR (150 MHz, D₂O) δ 179.9, 174.3, 170.3, 167.4, 130.6, 129.8, 129.6, 128.9, 128.5, 61.5, 56.4, 53.6, 47.5, 42.3, 40.6, 28.9, 24.6, 24.2, 22.4, 20.6;HRMS calcd for [C₂₁H₃₀N₄O₅ + H⁺] 419.2289, found: 419.2285.

### Example 4

### Synthesis of TP-4: H₂N-D-Chg-Pro-Gly-D-Leu-OH

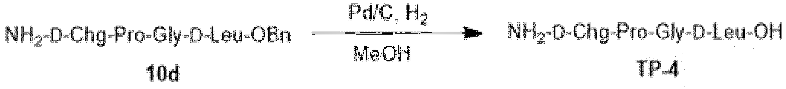

The NH₂-D-Chg-Pro-Gly-D-Leu-OBn **10d** (10.0 g, 19.4 mmol) synthetized above was dissolved in methanol (20 mL), transferred into a 100 mL autoclave, and then added with 500 mg of Pd/C (5%), and stirred for 6 hours with hydrogen having been introduced to 8 Mpa at room temperature. Pd/C was removed by filtration, and the filtrate was concentrated under reduced pressure. 7.94 g of tetrapeptide NH₂-D-Chg-Pro-Gly-D-Leu-OH (**TP-4**) was obtained as the product with a yield of 97%. White solid, Mp: 167―169 °C, [α]_{D}²⁰= ― 96.0 (c 1.0, H₂O); ¹H NMR (600 MHz, D₂O) δ 4.55 ― 4.46 (m, 1H), 4.33 ― 4.20 (m, 2H), 4.03 (d, *J=* 17.1 Hz, 1H), 3.93 ― 3.70 (m, 3H), 2.40 ― 2.32. (m, 1H), 2.13 ― 1.94 (m, 3H), 1.86 ― 1.57 (m, 7H), 1.37 ― 1.07 (m, 5H), 0.98 ― 0.86 (m, 6H); ¹³C NMR (150 MHz, D₂O) δ 179.6, 174.3, 170.4, 168.8, 61.1 , 56.6, 53.9, 53.4, 48.1, 42.4, 40.5, 38.4, 29.3, 28.5, 27.1, 25.1 , 24.5, 24.2, 22.4, 20.5; HRMS calcd for [C₂₁H₃₆N₄O₅ + H⁺] 425.2758, found 425.2748.

### Example 5

### Synthesis of TP-5: NH₂-D-Phe-Pro-Gly-D-Leu-OH

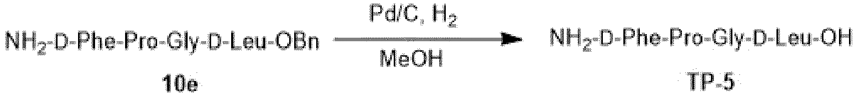

The NH₂-D-Phe-Pro-Gly-D-Leu-OBn **9e** (10.0 g, 19.1 mmol) synthetized above was dissolved in methanol (20 mL), transferred into a 100 mL autoclave, and then added with 500 mg of Pd/C (5%), and stirred for 6 hours with hydrogen having been introduced to 8 Mpa at room temperature. Pd/C was removed by filtration, and the filtrate was concentrated under reduced pressure. 7.7 g of tetrapeptide NH₂-D-Phe-Pro-Gly-D-Leu-OH (**TP-**5), white solid, was obtained as the product with a yield of 93%. White solid, Mp: 198 ― 200 °C; [α]_{D}²⁰ = ― 98.0 (c 1.0, H₂O); ¹H NMR (600 MHz, D₂O) 7.45 ― 7.39 (m, 3H), 7.35 (d, *J=* 6.9 Hz, 2H), 4.62 ― 4.57 (m, 1H), 4.37 (dd, *J =* 8.8, 4.6 Hz, 1H), 4.26 (dd, *J* = 10.5, 3.5 Hz, 1H), 4.01 *(d, J =* 17.1 Hz, 1H), 3.88 *(d, J =* 17.2 Hz, 1H), 3.64 ― 3.57 (m, 1H), 3.30 ― 3.18 (m, 2H), 2.86 ― 2.80(m, 1H), 2.16 ― 2.08 (m, 1H), 1.97 ― 1.85 (m, 2H), 1.73 ― 1.56 (m, 4H), 0.92 (dd, *J* = 28.0, 6.0 Hz, 6H); ¹³C NMR (150 MHz, D₂O) δ 179.8, 174.1, 170.3, 168.5, 133.3, 129.4, 129.1, 128.1, 61.0, 53.6, 53.0, 47.7, 42.4, 40.5, 36.3, 29.1, 24.5, 24.0, 22.4, 20.5; HRMS calcd for [C₂₂H₃₂N₄O₅ + H⁺] 433.2445, found 419.2439.

### Example 6

### Synthesis of TP-6: NH₂-D-Phe-Pro-Gly-D-Phe-OH

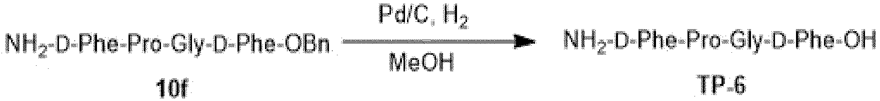

The NH₂-D-Phe-Pro-Gly-D-Phe-OBn **10f** (10.2 g, 18 mmol) synthetized above was dissolved in methanol (20 mL), transferred into a 100 mL autoclave, and then added with 500 mg of Pd/C (5%), and stirred for 6 hours with hydrogen having been introduced to 8 Mpa at room temperature. Pd/C was removed by filtration, and the filtrate was concentrated under reduced pressure. 7.8 g of tetrapeptide NH₂-D-Phe-Pro-Gly-D-Phe-OH (**TP-6**), a white solid, was obtained as the product with a yield of 92%. Mp 182.5―184.4 °C, [α]_{D}²⁰ = ― 50.0 (c 0.5, DMF); ¹H NMR (600 MHz, DMSO) δ 8.50 ― 8.16 (m, 2H), 7.41 ― 7.03 (m, 10H), 4.38 (m, 3H), 3.76 ― 3.64 (m, 1H), 3.50 ― 3.32 (m, 2H), 3.20 ― 2.79 (m, 4H), 1.87 ― 1.60 (m, 3H), 1.40 ― 1.26 (m, 1H), 0.98 (d, *J* = 6.5 Hz, 1H); ¹³C NMR (150 MHz, DMSO) δ 175.0, 171.4, 170.2, 168.3, 139.3, 136.0, 129.9, 129.7, 128.9, 128.3, 127.5, 126.2, 61.2, 52.9, 47.1, 42.1, 40.4 , 40.2, 40.1, 39.9, 39.7, 39.6, 38.8, 29.3, 24.4, 23.7; HRMS calcd for [C₂₅H₃₀N₄O₅ + H⁺] 467.2288, found: 467.2289..

### II. Application examples of a compound TP

### Example 7

In a clean 5 mL round-bottomed flask, an accurately weighed tetrapeptide catalyst **TP** (0.025 mmol) of the present disclosure, *N*-phenylmaleimide (87 mg, 0.5 mmol) and 1.0 mL of dried toluene were added, and redistilled isobutyraldehyde (92 µL, 1.0 mmol) was added under conditions of an ice bath and magnetic stirring. After returned to room temperature, the reaction solution was stirred continuously to react, and added with 3-4 drops of saturated ammonium chloride solution after the reaction was complete as detected by TLC. The resulting reaction solution was extracted with ethyl acetate (10 mL × 3), washed with a small amount of saturated brine, dried with anhydrous sodium sulfate, and concentrated under reduced pressure. The oily residue was purified by column chromatography with petroleum ether and ethyl acetate, to obtain a pure product.

The performance of 6 tetrapeptide catalysts **TP-1** - **TP-6** of the present disclosure to catalyze the asymmetric conjugate addition reaction of isobutyraldehyde with *N*-phenylmaleimide was evaluated. The results are shown in Table 1 below. It can be seen from the results that **TP-3** and **TP-5** have the highest asymmetric catalytic efficiency.

**Table 1. The asymmetric conjugate addition reaction of isobutyraldehyde with N-phenylmaleimide catalyzed by the tetrapeptide catalysts ^{a}**

| | | | | |
|---|---|---|---|---|
| 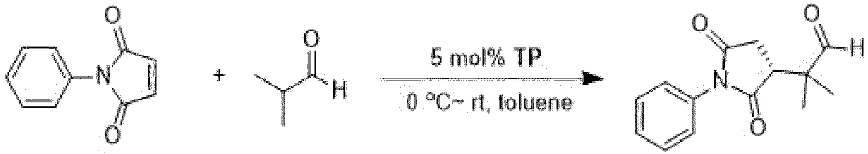 | | | | |

| entry | **TP** (5 mol%) | solvent | yield% | ee% |
|---|---|---|---|---|
| 1 | **TP-1** | toluene | 37 | 35 |
| 2 | **TP-2** | toluene | 29 | 72 |
| 3 | **TP-3** | toluene | 43 | 91 |
| 4 | **TP-4** | toluene | 38 | 41 |
| 5 | **TP-5** | toluene | 55 | 91 |
| 6 | **TP-6** | toluene | 43 | 85 |

| | | | | |
|---|---|---|---|---|
| *^{a}* Isolated yield, and ee were determined by chiral HPLC. | | | | |

### Example 8

Effects of solvents on the tetrapeptide **TP-5** to catalyze the asymmetric conjugate addition reaction of isobutyraldehyde with *N*-phenylmaleimide

In a clean 5 mL round-bottomed flask, an accurately weighed tetrapeptide catalyst **TP-5** (0.025 mmol, 10.8 mg), *N*-phenylmaleimide (87 mg, 0.5 mmol) and 1.0 mL of a dried and redistilled reaction solvent were added, and redistilled isobutyraldehyde (92 µL, 1.0 mmol) was added under conditions of an ice-water bath and magnetic stirring. After returned to room temperature, the reaction solution was stirred continuously to react, and added with 3-4 drops of saturated ammonium chloride solution after the reaction was complete as detected by TLC. The resulting reaction solution was extracted with ethyl acetate (10 mL × 3), washed with a small amount of saturated brine, dried with anhydrous sodium sulfate, and concentrated under reduced pressure. The oily residue was purified by column chromatography with petroleum ether and ethyl acetate, to obtain a pure product.

The results about effects of various solvents on the reaction yield and and ee value are shown in Table 2. These results show that: compared with that of toluene, when acetonitrile is used as a solvent, both the yield and ee value are significantly increased.

**Table 2. Effects of solvents on the tetrapeptide TP-5 to catalyze the asymmetric conjugate addition reaction of isobutyraldehyde and N-phenylmaleimide ^{a}**

| entry | solvent | hours | yield% | ee% |
|---|---|---|---|---|
| 1 | CH₂Cl₂ | 40 h | 80 | 99 |
| 2 | MeCN | 40 h | 95 | 98 |
| 3 | EtOH | 40 h | 91 | 88 |
| 4 | THF | 40 h | 90 | 97 |
| 5 | DMSO*^{b}* | -- | -- | --- |
| 6 | toluene | 72 h | 55 | 91 |

| | | | | |
|---|---|---|---|---|
| *^{a}* Isolated yield, and ee were determined by chiral HPLC. *^{b}* No reaction was detected. | | | | |

### Example 9

Investigation on effects of catalyst amounts on the catalytic efficiency of the asymmetric conjugate addition reaction

In a clean 5 mL round-bottomed flask, an accurately weighed tetrapeptide catalyst **TP-5** or **TP-3** (0.025 mmol), *N*-phenylmaleimide (87 mg, 0.5 mmol) and 1.0 mL of a dried and distilled reaction solvent were added, and distilled isobutyraldehyde (92 µL, 1.0 mmol) was added under conditions of an ice-water bath and magnetic stirring. After returned to room temperature, the reaction solution was stirred continuously to react, and added with 3-4 drops of saturated ammonium chloride solution after the reaction was complete as detected by TLC. The resulting reaction solution was extracted with ethyl acetate (10 mL × 3), washed with a small amount of saturated brine, dried with anhydrous sodium sulfate, and concentrated under reduced pressure. The oily residue was purified by column chromatography with petroleum ether and ethyl acetate, to obtain a pure product.

The reaction yields and ee values are shown in Table 3. The results show that using tetrapeptide **TP-3** at an amount of 2.5 mol% as the catalyst and acetonitrile as the solvent can obtain the best asymmetric catalytic effect.

**Table 3. Effects of amounts of the tetrapeptide used on the asymmetric conjugate addition reaction ^{a}**

| Entry | TP (mmol%) | solvent | hours | yield% | ee% |
|---|---|---|---|---|---|
| 1 | **TP-5** (5%) | MeCN | 40 h | 95 | 98 |
| 2 | **TP-5** (2.5%) | MeCN | 40 h | 95 | 98 |
| 3 | **TP-5** (1%) | MeCN | 40 h | 56 | 98 |
| 4 | **TP-3** (2.5%) | MeCN | 40 h | 98 | 99 |

| | | | | | |
|---|---|---|---|---|---|
| *^{a}* Isolated yield, and ee were determined by chiral HPLC. | | | | | |

### Example 10

Investigation on the range of applicable substrates in the tetrapeptide **TP-3** catalyze asymmetric conjugate addition reaction of an aliphatic aldehyde with a maleimide

In a clean 5 mL round-bottomed flask, 2.5% of accurately weighed tetrapeptide catalyst **TP-3** (0.0125 mmol, 5.3 mg), maleimide (0.5 mmol) and 1.0 mL of dried and distilled acetonitrile were added, and distilled aliphatic aldehyde (1.0 mmol) was added under conditions of an ice-water bath and magnetic stirring. After returned to room temperature, the reaction solution was stirred continuously to react, and added with 3-4 drops of saturated ammonium chloride solution after the reaction was complete as detected by TLC. The resulting reaction solution was extracted with ethyl acetate (10 mL × 3), washed with a small amount of saturated brine, dried with anhydrous sodium sulfate, and concentrated under reduced pressure. The oily residue was purified by column chromatography with petroleum ether and ethyl acetate, to obtain a pure product.

The results are shown in Table 4.

**Table 4. The asymmetric conjugate addition reaction of an aliphatic aldehyde with a maleimide catalyzed by the tetrapeptide TP-3 ^{a}**

| | | |
|---|---|---|
| 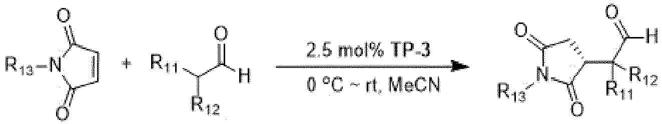 | | |
| 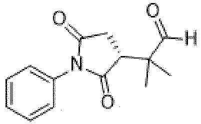 | 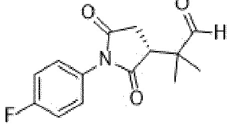 | 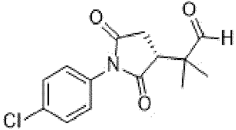 |
| P-1, y=96%, ee=99% | P-2, y=95%, ee=99% | P-3, y=90%, ee=98% |
| 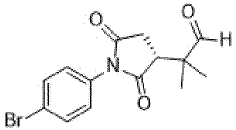 | 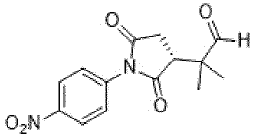 | 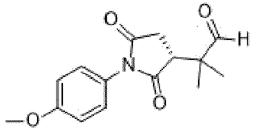 |
| P-4, y= 95%, ee=97% | P-5, y=80%, ee=99% | P-6, y=98%, ee=99% |
| 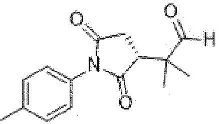 | 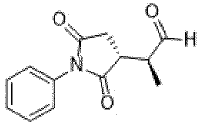 | 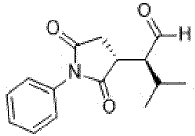 |
| P-7, y= 90%, ee=96% | P-8, y=88%, ee=99%, dr=74:26 | P-9, y=84%, ee=99%, dr=74:26 |
| 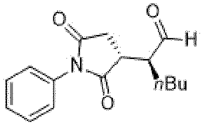 | 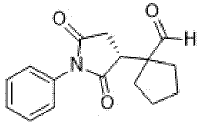 | 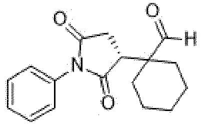 |
| P-10, y= 90%, ee=99% dr=93:7 | P-11, y=95%,ee=99% | P-12, y=96%, ee=99% |
| 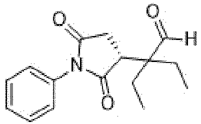 | 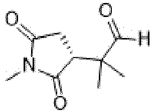 | 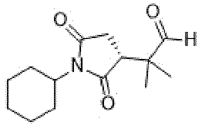 |
| P-13, y= 95%, ee=99% | P-14, y=90%, ee=96% | P-15, y=95%, ee=99% |
| 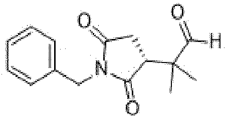 | 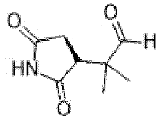 | |
| P-16, y=92%, ee=99% | P-17, y=85%, ee=92% | |

| | | |
|---|---|---|
| *^{a}* Isolated yield, and ee were determined by chiral HPLC. | | |

Characterizations of products **P-1** to **P-17** are as follows.

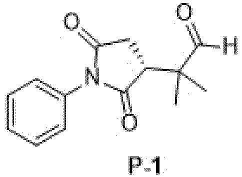

**P-1**, [α]_{D}²⁵ = ―6.0 *(c=* 1.0, CHCl₃); ¹H NMR (600 MHz, CDCl₃) δ 9.51 (s, 1H), 7.48-7.44 (m, 2H), 7.40 ― 7.36 (m, 1H), 7.29 ― 7.25 (m, 2H), 3.14 (dd, *J* = 9.6, 5.6 Hz, 1H), 2.96 (dd, *J* = 18.6, 9.6 Hz, 1H), 2.61 (dd, *J* = 18.4, 5.6 Hz, 1H), 1.32 (s, 3H), 1.28 (s, 3H).

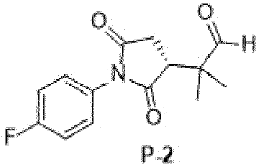

**P-2**, [α]_{D}²⁵ = +2.0 *(c=* 1.0, CHCl₃); ¹H NMR (600 MHz, CDCl₃) δ 9.49 (s, 1H), 7.28 ― 7.24 (m, 2H), 7.17 ― 7.12 (m, 2H), 3.11 (dd, *J* = 9.6, 5.6 Hz, 1H), 2.97 (dd, *J* = 18.3, 9.6 Hz, 1H), 2.61 (dd, *J* = 18.4, 5.6 Hz, 1H), 1.35 (s, 3H), 1.28 (s, 3H).

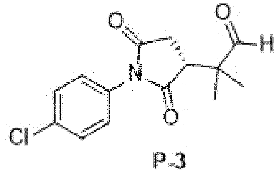

**P-3**, [α]_{D}²⁵ = +3.0 *(c=* 1.0, CHCl₃); ¹H NMR (600 MHz, CDCl₃) δ 9.49 (s, 1H), 7.49 ― 7.40 (m, 2H), 7.28 ― 7.22 (m, 2H), 3.11 (dd, *J* = 9.6, 5.6 Hz, 1H), 2.97 (dd, *J* = 18.3, 9.6 Hz, 1H), 2.62 (dd, *J* = 18.3, 5.6 Hz, 1H), 1.36 (s, 3H), 1.29 (s, 3H).

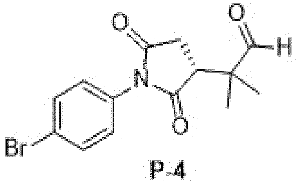

**P-4**, [α]_{D}²⁵ = +3.0 *(c=* 1.0, CHCl₃); ¹H NMR (600 MHz, CDCl₃) δ 9.49 (s, 1H), 7.62 ― 7.57 (m, 1H), 7.23 ― 7.15 (m, 2H), 3.11 (dd, *J* = 9.6, 5.4 Hz, 1H), 2.97 (dd, *J* = 18.4, 9.6 Hz, 1H), 2.62 (dd,*J*= 18.4, 5.6 Hz, 1H), 1.36 (s, 3H), 1.29 (s, 3H).

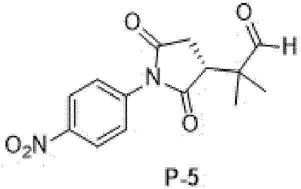

P-4, [α]_{D}²⁵ = +6.0 *(c=* 1.0, CHCl₃); ¹H NMR (600 MHz, CDCl₃) δ 9.46 (s, 1H), 8.35 ― 8.29 (m, 2H), 7.61 ― 7.54 (m, 2H), 3.10 (dd, *J* = 9.6, 5.4 Hz, 1H), 3.00 (dd, *J* = 18.6, 9.6 Hz, 1H), 2.65 (dd, *J* = 18.6, 5.4 Hz, 1H), 1.41 (s, 3H), 1.30 (s, 3H).

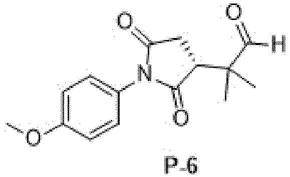

P-6, [α]_{D}²⁵ = +5.0 *(c=* 1.0, CHCl₃); ¹H NMR (600 MHz, CDCl₃) δ 9.52 (s, 1H), 7.21 ― 7.15 (m, 2H), 6.99 ― 6.95 (m, 2H), 3.82 (s, 3H), 3.13 (dd, *J* = 9.6, 5.4 Hz, 1H), 2.96 (dd, *J* = 18.4, 9.6 Hz, 1H), 2.60 (dd, *J* = 18.6, 5.4 Hz, 1H), 1.32 (s, 3H), 1.28 (s, 3H).

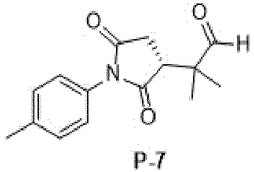

**P-7,** [α]_{D}²⁵ = +6.0 *(c=* 1.0, CHCl₃); ¹H NMR (600 MHz, CDCl₃) δ 9.52 (s, 1H), 7.27 (d, *J* = 7.2 Hz, 2H), 7.14 (d, *J* = 8.4 Hz, 2H), 3.14 (dd, *J* = 9.6, 5.4 Hz, 1H), 2.97 (dd, *J* = 18.4, 9.6 Hz, 1H), 2.61 (dd, *J* = 18.4, 5.5 Hz, 1H), 2.37 (s, 3H), 1.32 (s, 3H), 1.28 (s, 3H).

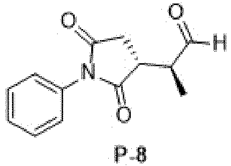

**P-8,** [α]_{D}²⁵ = +20.0 *(c=* 1.0, CHCl₃); ¹H NMR (600 MHz, CDCl₃) δ 9.63 (s, 1H), 7.48 (t, *J=* 7.8 Hz, 2H), 7.40 (t, *J=* 7.5 Hz, 1H), 7.34 ― 7.28 (m, 2H), 3.31-3.26 (m, 1H), 3.12 ― 3.06 (m, 1H), 2.93 (dd, *J* = 18.0, 9.7 Hz, 1H), 2.60 (dd, *J* = 18.0, 5.7 Hz, 1H), 1.40 (d, *J=* 7.8 Hz, 3H).

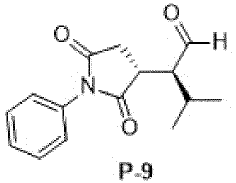

**P-9,** [α]_{D}²⁵ = +16.0 *(c=* 1.0, CHCl₃); ¹H NMR (600 MHz, CDCl₃) δ 9.78 (s, 1H), 7.50 ― 7.45 (m, 2H), 7.41 ― 7.36 (m, 1H), 7.31 ― 7.28 (m, 2H), 3.17 (dd, *J* = 7.2, 3.7 Hz, 1H), 3.11 ― 2.99 (m, 1H), 2.87 (dd, *J=* 18.0, 9.6 Hz, 1H), 2.73 (dd, *J=* 18.0, 6.0 Hz, 1H), 2.36―2.28 (m, 1H), 1.26 (d, *J* = 6.6 Hz, 3H), 1.10 (d, *J* = 6.6 Hz, 3H).

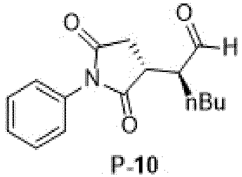

**P-10,** [α]_{D}²⁵ = +41.0 *(c=* 1.0, CHCl₃); ¹H NMR (600 MHz,CDCl₃) δ 9.75 (s, 1H), 7.47 (t, *J* = 6Hz, 2H), 7.41 ― 7.37 (m, 1H), 7.32-7.26 (m, 2H), 3.36 ― 3.32 (m, 1H), 3.03 ― 2.96 (m, 2H), 2.57 (dd, *J* = 18.0, 5.4 Hz, 1H), 1.94 ― 1.88 (m, 1H), 1.70 ― 1.62 (m, 1H), 1.51 ― 1.45 (m, 2H), 1.42 ― 1.36 (m, 2H), 0.93 (t, *J* = 6 Hz, 3H).

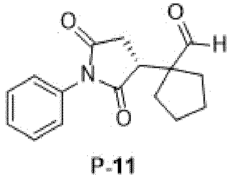

**P-11**, [α]_{D}²⁵ = ―40.0 *(c=* 1.0, CHCl₃); ¹H NMR (600 MHz, CDCl₃) δ 9.39 (s, 1H), 7.49-7.46 (m, 2H), 7.40 ― 7.37 (m, 1H), 7.34 ― 7.29 (m, 2H), 3.05 ― 2.94(m, 2H), 2.58 (dd, *J* = 18, 5.4 Hz, 1H), 2.37 ― 2.27 (m, 1H), 2.14 ― 2.03 (m, 2H), 1.88 ― 1.69 (m, 5H).

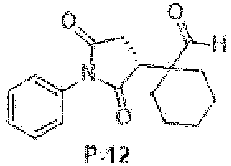

**P-12,** [α]_{D}²⁵ = ―5.0 *(c=* 1.0, CHCl₃);¹H NMR (600 MHz, CDCl₃) δ 9.55 (s, 1H), 7.47 (t, *J* = 7.7 Hz, 2H), 7.40 ― 7.38 (m, 1H), 7.31 ― 7.27 (m, 2H), 3.22 (dd, *J* = 9.0, 6.0 Hz, 1H), 2.87 (dd, *J* = 18.0, 9.6 Hz, 1H), 2.68 (dd, *J* = 18.2, 5.9 Hz, 1H), 1.99 ― 1.85 (m, 3H), 1.67 ― 1.49 (m, 6H).

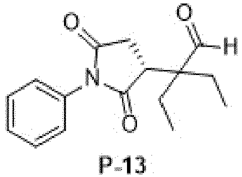

**P-13,** [α]_{D}²⁵ = +10.0 *(c=* 1.0, CHCl₃); ¹H NMR (600 MHz, CDCl₃) δ 9.63 (s, 1H), 7.47 (t, *J=* 7.8 Hz, 2H), 7.39 (t, *J=* 7.5 Hz, 1H), 7.29 ― 7.25 (m, 2H), 3.25 (dd, *J* = 9.6, 6.0 Hz, 1H), 2.97 (dd, *J* = 18.4, 9.6 Hz, 1H), 2.69 (dd, *J* = 18.4, 6.0 Hz, 1H), 2.02 ― 1.83 (m, 3H), 1.74 (dq, *J* = 15.0, 7.5 Hz, 1H), 1.03 ― 0.97 (m, 6H).

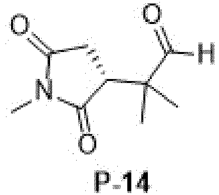

**P-14,** [α]_{D}²⁵ = ―9.0 *(c=* 1.0, CHCl₃); ¹H NMR (600 MHz, CDCl₃) δ 9.51 (s, 1H), 3.04 (dd, *J* = 9.6, 5.4 Hz, 1H), 2.99 (s, 3H), 2.82 (dd, *J* = 18.3, 9.4 Hz, 1H), 2.45 (dd, *J* = 18.3, 5.4 Hz, 1H), 1.22 (d, *J* = 6.4 Hz, 6H).

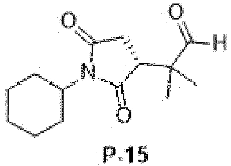

**P**-15, [α]_{D}²⁵ = ―11.0 *(c=* 1.0, CHCl₃); ¹H NMR (600 MHz, cdcl₃) δ 9.51 (s, 1H), 3.98 ― 3.91 (m, 1H), 2.97 (dd, *J* = 9.5, 5.2 Hz, 1H), 2.74 (dd, *J* = 18.3, 9.5 Hz, 1H), 2.37 (dd, *J* = 18.3, 5.2 Hz, 1H), 2.16 ― 2.08 m, 2H), 1.80 (d, *J* = 13.4 Hz, 2H), 1.66 ― 1.53 (m, 3H), 1.32 ― 1.23 (m, 2H), 1.23 ― 1.12 (m, 7H).

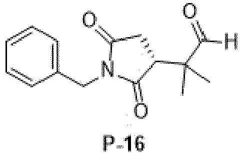

**P-16,** [α]_{D}²⁵ = -14.0 *(c=* 1.0, CHCl₃); ¹H NMR (600 MHz, CDCl₃) δ 9.48 (s, 1H), 7.37 ― 7.34 (m, 2H), 7.31 ― 7.28 (m, 2H), 4.65 (q, *J* = 14.4 Hz, 2H), 3.03 (dd, *J* = 9.4, 5.4 Hz, 1H), 2.81 (dd, *J* = 18.3, 9.4 Hz, 1H), 2.45 (dd, *J* = 18.3, 5.4 Hz, 1H), 1.16 (d, *J* = 2.3 Hz, 6H).

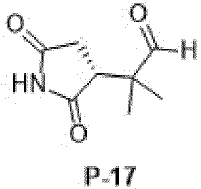

**P-17,** [α]_{D}²⁵ = +5.0 *(c=* 1.0, CHCl₃); ¹H NMR (600 MHz, CDCl₃) δ 9.46 (s, 1H), 8.81 (s, 1H), 3.07 (dd, *J* = 9.6, 5.4 Hz, 1H), 2.83 (dd, *J* = 18.4, 9.6 Hz, 1H), 2.48 (dd, *J* = 18.4, 5.4 Hz, 1H), 1.21 (d, *J* = 6.9 Hz, 6H).

The above description of the examples is only used to facilitate understanding of the method and core concept of the present disclosure. It should be noted that it is possible for those skilled in the art to make various improvements and modifications without departing from the principle of the present disclosure, and these improvements and modifications should fall within the scope of protection of the claims.

## Claims

1. A tetrapeptide compound **TP** represented by Formula 1, 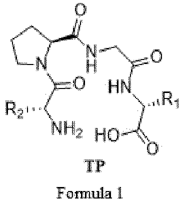 wherein R₁ and R₂ are independently selected from any one of C₁-C₆ straight-chain alkyl, branched-chain alkyl, cycloalkyl, hydroxy-substituted alkyl, sulfhydryl-substituted alkyl, methylthio-substituted alkyl, amino-substituted alkyl, guanidyl-substituted alkyl, aryl, arylmethyl or heteroaryl.

2. The tetrapeptide compound **TP** according to claim 1, wherein R₁ is selected from any C₁-C₆ straight-chain alkyl, C₃-C₁₅ branched-chain alkyl, C₃-C₈ cycloalkyl, hydroxyl-substituted C₁-C₆ alkyl, sulfhydryl-substituted C₁-C₆ alkyl, methylthio-substituted C₁-C₆ alkyl, amino-substituted C₁-C₆ alkyl, guanidyl-substituted C₁-C₆ alkyl, C₆-C₃₀ aryl, C₆-C₃₀ arylmethyl or C₅-C₁₅ heteroaryl; and
R₂ is selected from any C₁-C₆ straight-chain alkyl, C₃-C₁₅ branched-chain alkyl, C₃-C₈ cycloalkyl, hydroxyl-substituted C₁-C₆ alkyl, sulfhydryl substituted C₁-C₆ alkyl, methylthio-substituted C₁-C₆ alkyl, amino-substituted C₁-C₆ alkyl, guanidyl-substituted C₁-C₆ alkyl, C₆-C₃₀ aryl, C₆-C₃₀ arylmethyl or C₅-C₁₅ heteroaryl.

3. The tetrapeptide compound **TP** according to claim 1, wherein R₁ is methyl, ethyl, *n*-propyl, isopropyl, *n*-butyl, isobutyl, *t*-butyl, *n*-pentyl, isoamyl, *n*-hexyl, cyclopropyl, cyclobutyl, cyclopentyl, cyclohexyl, cycloheptyl, hydroxymethyl, 2-hydroxyethyl, 1-hydroxyethyl (MeCH(OH)-), aminomethyl, 2-aminoethyl, 3-amino n-propyl, 4-amino *n*-butyl, mercaptomethyl, 2-mercaptoethyl, methylthiomethyl, 2-methylthioethyl, phenyl, naphthyl, anthryl, phenanthryl, furyl, pyridyl, pyranyl, piperidinyl, phenylmethyl, naphthylmethyl or anthrylmethyl; and
R₂ is methyl, ethyl, *n*-propyl, isopropyl, *n*-butyl, isobutyl, *t*-butyl, *n*-pentyl, isoamyl, *n*-hexyl, cyclopropyl, cyclobutyl, cyclopentyl, cyclohexyl, cycloheptyl, hydroxymethyl, 2-hydroxyethyl, 1-hydroxyethyl (MeCH(OH)-), aminomethyl, 2-aminoethyl, 3-amino n-propyl, 4-amino *n*-butyl, mercaptomethyl, 2-mercaptoethyl, methylthiomethyl, 2-methylthioethyl, phenyl, naphthyl, anthryl, phenanthryl, furyl, pyridyl, pyranyl, piperidinyl, phenylmethyl, naphthylmethyl or anthrylmethyl.

4. The tetrapeptide compound **TP** according to claim 1, wherein in the tetrapeptide compound **TP** having the structure of Formula (I),
R₁ is *i*-Bu, and R₂ is *i*-Pr, that is, the tetrapeptide compound **TP** has an amino acid sequence of H₂N-D-Val-Pro-Gly-D-Leu-OH which is named **TP-1;**
R₁ is *i*-Bu, and R₂ is *t*-Bu, that is, the tetrapeptide compound **TP** has an amino acid sequence of H₂N-D-Tle-Pro-Gly-D-Leu-OH which is named **TP-2;**
R₁ is *i*-Bu, and R₂ is Ph, that is, the tetrapeptide compound **TP** has an amino acid sequence of H₂N-D-Phg-Pro-Gly-D-Leu-OH which is named **TP-3;**
R₁ is *i*-Bu, and R₂ is c-hex, that is, the tetrapeptide compound **TP** has an amino acid sequence of H₂N-D-Chg-Pro-Gly-D-Leu-OH which is named **TP-4;**
R₁ is *i*-Bu, and R₂ is Bn, that is, the tetrapeptide compound **TP** has an amino acid sequence of H₂N-D-Phe-Pro-Gly-D-Leu-OH which is named **TP-5;** and
R₁ is Bn, and R₂ is Bn, that is, the tetrapeptide compound **TP** has an amino acid sequence of H₂N-D-Phe-Pro-Gly-D-Phe-OH which is named **TP-6.**

5. A method of preparing the tetrapeptide compound **TP** represented by Formula 1 according to claim 1, comprising:
1) mixing and reacting a product **7** with an amino acid **8,** to obtain a product **9;** 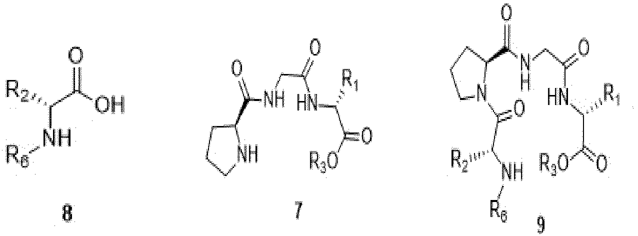
wherein R₁ and R₂ are independently any one selected from any C₁-C₆ straight-chain alkyl, branched-chain alkyl, cycloalkyl, hydroxy-substituted alkyl, sulfhydryl-substituted alkyl, methylthio-substituted alkyl, amino-substituted alkyl, guanidyl-substituted alkyl, aryl, arylmethyl or heteroaryl;
R₃ is arylmethyl;
R₆ is selected from tert-butyloxycarbonyl, benzyloxycarbonyl or fluorenylmethoxycarbonyl; and
2) converting the product **9** into a tetrapeptide compound **TP** represented by Formula 1;

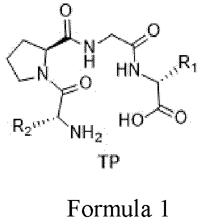

6. The preparation method according to claim 5, wherein the product 7 is prepared by a method comprising steps of:
A-1) reacting a product **4** with a proline **5**, to obtain a product **6**; 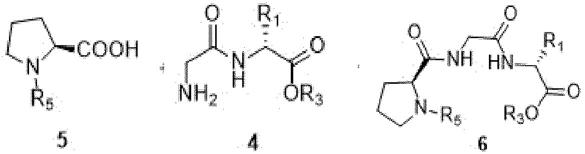 wherein R₁ is any one selected from any C₁-C₆ straight-chain alkyl, branched-chain alkyl, cycloalkyl, hydroxy-substituted alkyl, sulfhydryl-substituted alkyl, methylthio-substituted alkyl, amino-substituted alkyl, guanidyl-substituted alkyl, aryl, arylmethyl or heteroaryl;
R₃ is arylmethyl;
R₅ is selected from tert-butyloxycarbonyl, benzyloxycarbonyl or fluorenylmethoxycarbonyl; and
A-2) converting the product **6** into the product **7.**

7. The preparation method according to claim 6, wherein the product 4 is prepared by a method comprising steps of:
B-1) mixing and reacting a glycine 1 with an amino acid **2,** to obtain a product **3;** 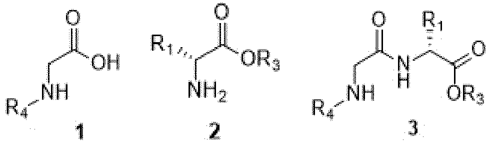 wherein R₁ is any one selected from any C₁-C₆ straight-chain alkyl, branched-chain alkyl, cycloalkyl, hydroxy-substituted alkyl, sulfhydryl-substituted alkyl, methylthio-substituted alkyl, amino-substituted alkyl, guanidyl-substituted alkyl, aryl, arylmethyl or heteroaryl;
R₃ is arylmethyl;
R₄ and R₅ are independently selected from tert-butyloxycarbonyl, benzyloxycarbonyl or fluorenylmethoxycarbonyl; and
B-2) converting the product **3** into the product **4.**

8. The preparation method according to claim 5, wherein the step 2) consists of: deprotecing the product **9,** to obtain a product **10;** 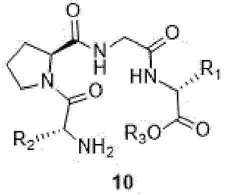
wherein R₁ and R₂ are independently any one selected from any C₁-C₆ straight-chain alkyl, branched-chain alkyl, cycloalkyl, hydroxy-substituted alkyl, sulfhydryl-substituted alkyl, methylthio-substituted alkyl, amino-substituted alkyl, guanidyl-substituted alkyl, aryl, arylmethyl or heteroaryl;
R₃ is arylmethyl;
R₆ is selected from tert-butyloxycarbonyl, benzyloxycarbonyl or fluorenylmethoxycarbonyl; and
subjecting the product **10** to a catalytic hydrogenation reaction, to obtain the tetrapeptide compound **TP** represented by Formula 1.

9. The preparation method according to claim 5, wherein the preparation method of the tetrapeptide compound **TP** represented by Formula 1 is performed by: carring out reactions as shown in Formula 2, Formula 3, Formula 4 and Formula 5, that is: 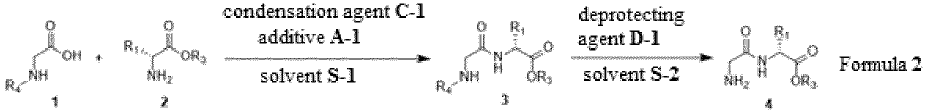 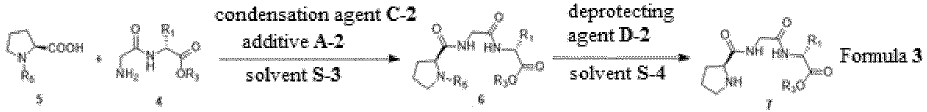 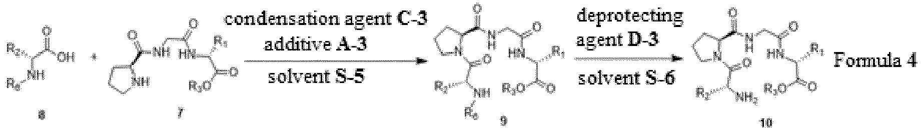 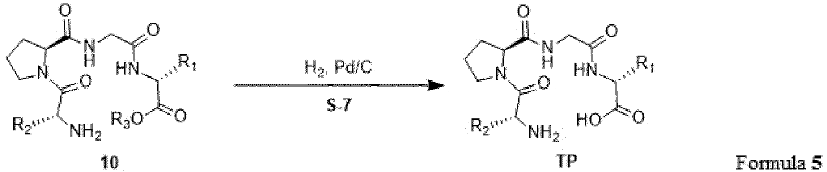
adding a glycine **1,** an amino acid **2,** a condensation agent **C-1** and an additive **A-1** into a solvent **S-1** and stirring to react to obtain a product **3,** then adding the product **3** and a deprotecting agent **D-1** into a solvent **S-2** to react to obtain a product **4;**
adding the product **4,** a proline **5,** a condensation agent **C-2** and an additive **A-2** into a solvent **S-3** and stirring to react to obtain a product **6,** then adding the product **6** and a deprotecting agent **D-2** into a solvent **S-4** and stirring to react to obtain a product 7;
adding the product **7,** an amino acid **8,** a condensation agent **C-3** and an additive **A-3** into a solvent **S-5** and stirring to react to obtain a product **9,** then adding the product **9** and deprotecting agent **D-3** into a solvent **S-6** and stirring to react to obtain a product 10; and
adding the product **10** and a catalyst Pd/C into a solvent **S-7** to react with hydrogen gas introduced at a pressure of 1-30 Mpa, to obtain a final product of tetrapeptide **TP,** wherein:
R₃ is arylmethyl;
R₄, R₅ and R₆ are any one of Boc (tert-butyloxycarbonyl), Cbz (benzyloxycarbonyl) or Fmoc (fluorenylmethoxycarbonyl);
condensation agents **C-1**, **C-2** and **C-3** are any one of carbodiimides or alkoxyformyl chlorides R₇OCOCl, and R₇ is any C₁-C₄ straight-chain alkyl or C₁-C₄ branched-chain alkyl;
additives **A-1, A-2,** and **A-3** refer to R₈R₉R₁₀N or NMM (N-methylmorpholine) or TMEDA (4-dimethylaminopyridine) or HOBt (1-hydroxybenzotriazole), and R₈, R₉ and R₁₀ are any C₁-C₄ straight-chain alkyl or C₁-C₄ branched-chain alkyl;
solvents **S-1, S-2, S-3, S-4, S-5,** and **S-6** are any one of toluene, benzene, dichloromethane, dichloroethane, ethyl ether, tetrahydrofuran, ethylene glycol dimethyl ether, methyl tert-butyl ether, dioxane, ethyl acetate, methyl acetate, acetonitrile or propionitrile, or a combination thereof, and solvent S-7 is any one of methanol, ethanol, propanol or butanol, or a combination thereof; and
deprotecting agents **D-1, D-2** and **D-3** are any one of methanesulfonic acid, trifluoromethanesulfonic acid, benzenesulfonic acid, *para*-toluenesulfonic acid, trifluoroacetic acid, trichloroacetic acid, hydrochloric acid, sulfuric acid, piperidine, morpholine, tetrahydropyrrole, dihydropyrrole, pyrrole, diethylamine, dipropylamine, dibutylamine, diisopropylamine or diisobutylamine.

10. The preparation method according to claim 9, wherein:
R₃ is benzyl;
R₄, R₅ and R₆ are independently selected from Boc or Cbz;
condensation agents **C-1**, **C-2** and **C-3** are independently selected from DCC (N,N'-dicyclohexylcarbodiimide) or DIC (N,N'-diisopropylcarbodiimide) or an alkoxyformyl chloride R₇OCOCl,
wherein R₇ is Et or *i*-Pr or *i*-Bu;
additives **A-1, A-2** and **A-3** are independently any one selected from TEA (triethylamine), DIPEA (diisopropylethylamine), NMM or HOBt;
solvents **S-1, S-2, S-3, S-4, S-5** and **S-6** are independently selected from dichloromethane or tetrahydrofuran, and solvent **S-7** is selected from methanol or ethanol;
deprotecting agents **D-1, D-2** and **D-3** are independently selected from methanesulfonic acid, or trifluoromethanesulfonic acid or trifluoroacetic acid.

11. The preparation method according to claim 9, wherein
R₃ is benzyl;
R₄, R₅ and R₆ are independently Boc;
condensation agents **C-1**, **C-2** and **C-3** are independently selected from DCC, DIC or alkoxyformyl chloride R₇OCOCl, and R₇ is selected from Et or *i*-Pr or *i*-Bu;
additives **A-1, A-2** and **A-3** are independently any one selected from TEA, DIPEA, NMM or HOBt;
solvents **S-1, S-2, S-3, S-4, S-5,** and **S-6** are independently selected from dichloromethane or tetrahydrofuran, and solvent **S-7** is methanol;
deprotecting agents **D-1, D-2** and **D-3** are independently trifluoroacetic acid; and the hydrogen gas is of a pressure of 8 Mpa.

12. A catalyst for catalyzing an asymmetric conjugate addition reaction of an alipatic aldehyde with a maleimide, wherein the catalyst is the tetrapeptide compound **TP** represented by Formula 1 according to any one of claims 1-4.

13. An intermediate compound for use in preparation of a compound **TP** represented by Formula 1, which is the compound **6** as shown in Formula 3.

14. An intermediate compound for use in preparation of a compound **TP** represented by Formula 1, which is the compound **7** as shown in Formula 3.

15. An intermediate compound for use in preparation of a compound **TP** represented by Formula 1, which is the compound **9** as shown in Formula 4.

16. An intermediate compound for use in preparation of a compound **TP** represented by Formula 1, which is the compound **10** as shown in Formula 4.
